# EUROPEAN PATENT APPLICATION

(11) **EP 2 857 839 A1**
(43) Date of publication of application: **08.04.2015**
(21) Application number: 13186847.3
(22) Date of filing: 01.10.2013
(51) Int. Cl.: G01N 33/564, G01N 33/574

(54) **Breast cancer diagnostic method and means**

(71) Applicant: AIT Austrian Institute of Technology GmbH, 1220 Wien (AT)
(72) Inventor: Weinhäusel, Andreas, 7311 Neckenmarkt (AT); Gyurjan, Istvan, 1220 Vienna (AT)
(74) Representative: Sonn & Partner Patentanwälte

(57) **Abstract**

The present invention discloses a method of diagnosing breast cancer by using specific markers from a set, having diagnostic power for breast cancer diagnosis and distinguishing breast cancer types in diverse samples.

## Description

The present invention relates to cancer diagnostic methods and means therefor.

Neoplasms and cancer are abnormal growths of cells. Cancer cells rapidly reproduce despite restriction of space, nutrients shared by other cells, or signals sent from the body to stop reproduction. Cancer cells are often shaped differently from healthy cells, do not function properly, and can spread into many areas of the body. Abnormal growths of tissue, called tumors, are clusters of cells that are capable of growing and dividing uncontrollably. Tumors can be benign (noncancerous) or malignant (cancerous). Benign tumors tend to grow slowly and do not spread. Malignant tumors can grow rapidly, invade and destroy nearby normal tissues, and spread throughout the body. Malignant cancers can be both locally invasive and metastatic. Locally invasive cancers can invade the tissues surrounding it by sending out "fingers" of cancerous cells into the normal tissue. Metastatic cancers can send cells into other tissues in the body, which may be distant from the original tumor. Cancers are classified according to the kind of fluid or tissue from which they originate, or according to the location in the body where they first developed. All of these parameters can effectively have an influence on the cancer characteristics, development and progression and subsequently also cancer treatment. Therefore, reliable methods to classify a cancer state or cancer type, taking diverse parameters into consideration is desired.

In cancer-patients serum-antibody profiles change as well as autoantibodies against the cancerous tissue are generated. Those profile-changes are highly potential of tumor associated antigens as markers for early diagnosis of cancer. The immunogenicity of tumor associated antigens are conferred to mutated amino acid sequences, which expose an altered non-self epitope. Other explanations for its immunogenicity include alternative splicing, expression of embryonic proteins in adulthood, deregulation of apoptotic or necrotic processes and abnormal cellular localizations (e.g. nuclear proteins being secreted). Other explanations are also implicated of this immunogenicity, including alternative splicing, expression of embryonic proteins in adulthood, deregulation of apoptotic or necrotic processes, abnormal cellular localizations (e.g. nuclear proteins being secreted). Examples of epitopes of the tumour-restricted antigens, encoded by intron sequences (i.e. partially unspliced RNA were translated) have been shown to make the tumor associated antigen highly immunogenic. However until today technical prerequisites performing an efficient marker screen were lacking.

The object of the present invention is therefore to provide improved marker sequences and the diagnostic use thereof for the treatment of breast cancer.

The provision of specific markers permits a reliable diagnosis and stratification of patients with breast cancer, in particular by means of a protein biochip.

The invention therefore relates to the use of marker proteins for the diagnosis of breast cancer, wherein at least one marker protein is selected from the marker proteins of List 1. **List 1:** Marker proteins given by their Protein Symbol. FURIN, GTF2IRD1, LCP1, MAGED2, MEGF8, NARG2, PIN1, PRPF31, RASGRP2, SNRNP35, STX18, TNXB, TXN2, VTI1B, CTBP2, E4F1, FIS1 (includes EG:288584), HNRNPF, HNRNPUL1, MYO9B, NEDD4L, NOP58, OS9, PHACTR2, RSPH10B/RSPH10B2, SCAP, SERBP1, SORBS2, STXBP3, USP12, ARID5A, ATAD3A/ATAD3B, ATP5SL, BCL3, BSDC1, C12orf32, C1orf144, CCDC64, CDK11A/CDK11B, CDK5RAP2, CDT1, CELF5, DDX19B, DDX24, EIF3G, FAM171B, FAM208A, FKBP8, GIPC1, GMFG, HADH, HNRNPA1, HSPG2, HTT, ISG15, IST1 (includes EG:307833), KANSL2, KIF22, MED12, MED23, MGA, MTUS1, MYH14, NFKBID, NUMA1, PNPT1, PPM1G, RAB11FIP3, RAD21, RRBP1, SETD4, TAOK1, TPM3, UBA1, VIM, VPS8 (includes EG:209018), ZC3H7B, CHKB, EFR3A, MAPKAPK2, MKI67IP, P4HTM (includes EG:301008), PSMB8, TUBA1B, ZC3H7A, ABAT, ACOT9, ACTN4, APBB1, BCAM, BIN1, CCT5, CPE, DNAJC10, HMGB2, IRF1 (includes EG:16362), KRT8, NRXN2, PITPNC1, PPP2R5D, PRRT1, SAMD14, SDCBP, SNRNP200, SPAG9, WDR47, ZFP14, ZFYVE28, ZNF317, ZNFX1, AEBP1, ANXA6, APOH, BOD1L1, BRPF1, C12orf45, CCDC94, CDK13, CENPF, CEP70, CHD3, CLTC, CSRNP1, EEFSEC, FAM134A, FBXO34, FCHO1, FLII, FNTB, FOXL1, GABARAPL2, GDAP1L1, HLA-C, HSP90AB1, KAT7, KHDRBS1, KLHDC3, LRP1 (includes EG:16971), MAF1 (includes EG:315093), MAPK8IP2, NPM1, NT5C3L, PFAS, PLCG1, POGK, POGZ, PPIP5K1, PRDX1, PREPL, PRKD2, PRRC2C, PTPN23, PXN, QARS, RAB5C, RBM25, RBM5, SLC7A5, SND1, SNX5, SPTBN1, SRA1, TAF4B, TIAF1, TRADD, TSC2, TXLNA, U2AF1, UBE2B, UBR5, URI1, VPS37B, ADAMTS16, AKR1C4, AKT1, AP2A1, ARCN1, ARHGAP1, CCND1, CD81, CD97, CDC25B, CINP, CRAT, DOK2, EHD4, FGB, FLNA, FTL, FUT8, GBAS, HARS, HIVEP1, HOOK2, LRRC47, LRSAM1, NAGLU, PACS1, PLCE1, RPS18, SH3BGRL3, STAB1, TXLNG2P, VPS72 (includes EG:100001285), ACIN1, ACTR3, ADH5 (includes EG:100145871), AMN1 (includes EG:196394), AMT, ATN1, ATRX, AURKAIP1, CISH, CLCN2, CPNE1, CYLD, DENND1C, EIF3J, EIF3L, EZR, FBXO6, GOLM1, HLA-A, HLA-B, KPNB1, LAMC1, MAPK8IP3, NAP1L1, NDC80, NUP85 (includes EG:287830), OLFM2, OTUB1, PDCD6IP, POLR2H, PSMC4, PTK2B, PTPN7, RAC2, RAD9A, RAN, RPS17/RPS17L, SCG5, SCO1, SNX6, SRPR, STIP1, SUPT5H, TMOD3, AARS, AMBRA1, ANTXR1, ARHGAP44, ATXN1, BAZ1B, C12orf35, CAMSAP1, CHMP1B, CTSH, DDX39A, DFFA, EEF1D, FBRS, FBXL5, GART, GGA3, GLUL, GMPPB, GNAI2, HSPA1A/HSPA1B, HSPB1, HUWE1, IGF2R, LUC7L3, MAP3K3, MCM3AP, MGEA5, MINK1, MTA2, MUC2 (includes EG:4583), NISCH, NLE1, PCDH7, PDXK, PJA2, PLXNA1, PSMD13, PTPRF, RABEPK, RNF213, RPLP0P2, RPS6KB2, SBF1, SEC13, SEPT1, SEPT6, SLC25A6, SNX4, SPNS2, STAT1, TAX1BP1, THAP7, TMSB10/TMSB4X, TNFSF13, TPR, TRIP12, TYK2, UBA52, UBR4, UQCRC1, ACAT2, ADAMTS10, AFMID, AGAP6 (includes others), APBB1IP, ARHGEF11, ATIC, BCL11A, BEX4, C8orf37, CACNB3, CLU, CORO2B, CPNE6, EGR1, LCK, MAP3K4, MCM7, MMP14, NEFM, NOC2L, NONO, PCDH9, PLEKHH3, PMS2, PTPRA, RARS, RGS2 (includes EG:19735), RNF146, RPL10, SF3A3, SH3BP1, STMN4, STRN4, TPM2, TSEN2, TTYH1, VASP, VAV2, WARS, WDR6, WDTC1, ZC3H13, ZFP2, ZNF7, ABCA11P, AKAP9, ARID4B, ATG16L1, ATXN2, C17orf56, CCDC149, CCL28, CEP57, CIC, CNTROB, COL3A1, DMPK, FLOT1, FOXP3, GLYR1, GNAS, GOLGA7, HMOX2, IGLC1, KIAA1244, LPXN, MORF4L1, NCOA4, NRG3, NXPH3, PARD3, PDZD4, PRKCD, RASGRP1, RCOR2, RRM1, SAP18, SFN, SSBP2, TAF7, THOP1, TNFRSF6B, TRIM44, TXNDC5, UTY, WDR33, WDR73, ZNF638, A2M, ABI1, AIDA, ANXA11, ARAP1, ARHGEF40, ARL8A, BZW2, C21orf2, CHD1L, DBNL, EIF4G1, ERP29, EVPL, FAM192A, GOLGA2, HK1, HNRNPA3, HTATIP2, LARP1, LSP1 (includes EG:16985), MICAL3, PCGF2, PKP3, PLCB3, POLR3E, PO-TEE/POTEF, PSMB3, R3HDM4, RIPK1, SAFB2, SMAP2, SMCR8, SPHK2, SURF4, TNRC18, TRAFD1, WASH1/WASH5P, ZNF839, ACTR1B, APEX1, ATP5D, BZRAP1, C17orf70, C19orf25, CDC37, CNTD1, CTSD, DHX16, DHX9, FIGNL1, FZR1, KCNJ14, KLF5, LOC440354, MAPRE1, MYO9A, P4HB, PEF1, PIP5K1A, POLI, RCBTB1, RSL24D1, SIRT2, SPOCK2, STAT6, STK10, SURF6, TALDO1, UNC45A, ZNF202, AMICA1, ARF5, CECR5, CSF1R, CUEDC2, CUL1, GON4L, ITFG3, LCMT2, MCM5, METTL3, POLR2L, PSME4, RNF34, STAT4, WAC, XRCC1 (includes EG:22594), BIN3, EHMT1, KIFC3, LRBA, NAPA, NEU1, PLBD1, PLOD1, PLXNA3, PPIF, PRDX6, TSC22D3, WDR75, CBX4, CCDC51, CCNL2, CUL7, DCAF6, DSP, ENTPD6, ETFA, EXOSC10, EZH1, HEXDC, HMGN2, HSP90B1, IGHG1, IK, ILF3, IMMT, IMP4, KAT2A, KCMF1, LDHB (includes EG:3945), LOC284023, LTBP3, MYCL1, NEFL, NUDT16L1, PLEKHA4, PLEKHO1, PPID, PPP1R9B, PSMB6, PTPRE, RCL1, RECQL4, RPL37A, RPL7, SCAF1, SMARCC2, STX11, SWI5, TRO, VIMP, AIP, BNIP1, BTBD7, C3orf19, EIF4B, FAM69B, HNRNPA0, HNRNPK, HNRPDL, KCNK5, LGALS3, LONP1, METTL17, MSLN, PDE2A, PUF60, RNF219, RSPRY1, SAT2, SSRP1, TBC1D9B, ZSWIM4, AGAP2, AKAP17A, ANGPTL2, AP2M1, ARL4D, CANX, CELSR1, D2HGDH, HIST3H2A, HNRNPH1, HSPA9, INPPL1, NRBP2, NUP93, PA2G4, PGD, PGRMC1, POLR2B, RBM10, SLU7 (includes EG:10569), SPTAN1, ST3GAL3, STOML2, TF, TTC3, ZNF132, ZNF300, ZNF431.

Although the detection of a single marker can be sufficient to indicate a risk for breast cancer, it is preferred to use more than one marker, e.g. 2, 3, 4, 5, 6, 7, 8, 9, 10, 11, 12 or more markers in combination, especially if combined with statistical analysis. From a diagnostic point of view, a single autoantigen based diagnosis can be improved by increasing sensitivity and specificity by using a panel of markers where multiple auto-antibodies are being detected simultaneously. Particular preferred combinations are of markers within one of the marker lists 2 to 20 as identified further herein.

The inventive markers are suitable protein antigens that are overexpressed in tumor and can be used to either identify cancerous tissue or to differentiate between histological breast cancer types. The markers usually cause an antibody reaction in a patient. Therefore the most convenient method to detect the presence of these markers in a patient is to detect antibodies against these marker proteins in a sample from the patient, especially a body fluid sample, such as blood, plasma or serum.

To detect an antibody in a sample it is possible to use marker proteins as binding agents and subsequently to detect bound antibodies. It is not necessary to use the entire marker proteins but it is sufficient to use antigenic fragments that are bound by the antibodies. "Antigenic fragment" herein relates to a fragment of the marker protein that causes an immune reaction against said marker protein in a human. Preferred antigenic fragments of any one of the inventive marker proteins are the fragments of the clones as identified by the UniqueID. Such antigenic fragments may be antigenic in a plurality of humans, such as at least 5, or at least 10 individuals.

"Diagnosis" for the purposes of this invention means the positive determination of breast cancer by means of the marker proteins according to the invention as well as the assignment of the patients to breast cancer. The term "diagnosis" covers medical diagnostics and examinations in this regard, in particular in-vitro diagnostics and laboratory diagnostics, likewise proteomics and peptide blotting. Further tests can be necessary to be sure and to exclude other diseases. The term "diagnosis" therefore likewise covers the differential diagnosis of breast cancer by means of the marker proteins according to the invention and the risk or prognosis of breast cancer.

Specific indications that can be identified with one or more of the inventive markers are breast cancer and in particular also histological types of breast cancer. Particular differentiations that can be made with the inventive markers and methods are distinguishing 1) healthy conditions vs. benign tumor, 2) healthy conditions vs. malign tumor, 3) benign tumor vs. malign tumor, 4) healthy conditions vs. hereditary breast cancer, and 5) malign tumor vs. hereditary breast cancer.

The invention thus also relates to a surgical method comprising detecting cancer according to the present invention and removing said cancer.

In particular the inventive method may comprise detecting breast cancer when distinguishing healthy conditions vs. benign tumor, healthy conditions vs. malign tumor, benign tumor vs. malign tumor, healthy conditions vs. hereditary breast cancer, and malign tumor vs. hereditary breast cancer. A positive result in distinguishing said indications can prompt a further cancer test, in particular more invasive tests than a blood test such as an biopsy.

The inventive markers are preferably grouped in sets of high distinctive value. Some sets excel at diagnosing or distinguishing 1, 2, 3, 4, or 5 of the above identified indications.

Preferred markers are of List 2, which comprise markers for all of the above indications 1) to 5).

**List 2:** Preferred marker protein set, suitable for multiple analytic distinctions; Proteins are identified by protein symbol: ACAT2, ACTN4, ADAMTS10, AMBRA1, ANXA6, ARHGAP44, ATIC, CCND1, CDT1, CORO2B, CTSH, DFFA, E4F1, EHMT1, EIF3G, FAM208A, FLNA, GLUL, HLA-C, HNRNPA1, HNRNPUL1, HSPA1A/HSPA1B, IGF2R, IST1 (includes EG:307833), KANSL2, KIFC3, LCK, MAP3K4, MCM3AP, MED23, MGA, MYH14, NAPA, NEFM, NISCH, NUMA1, PACS1, PJA2, PLEKHH3, PLOD1, PLXNA3, PNPT1, PPM1G, PRDX6, PRPF31, PTPRA, PTPRE, PTPRF, RAB11FIP3, RAD21, RNF213, RNF34, RPLP0P2, SBF1, SEPT1, SLC7A5, SORBS2, TAOK1, THAP7, TRIP12, VIM, VPS72 (includes EG:100001285), ZNF7.

In particular embodiments, the invention provides the method of diagnosing breast cancer or the risk of breast cancer in a patient by detecting at least 2 of the marker proteins selected from the markers of List 2 in a patient comprising the step of detecting antibodies binding said marker proteins, detecting said marker proteins or antigenic fragments thereof in a sample of the patient. Also provided is a method of diagnosing breast cancer or the risk of breast cancer in a patient by detecting at least 20%, preferably at least 30%, especially preferred at least 40%, at least 50%, at least 60%, at least 70%, at least 80% at least 90% or all of the marker proteins selected from the markers of List 2 in a patient comprising the step of detecting antibodies binding said marker proteins, detecting said marker proteins or antigenic fragments thereof in a sample of the patient.

Further preferred marker sets according to the present invention are provided in example 7 as lists 3 to 20. Thus the present invention also provides the method of diagnosing breast cancer or the risk of breast cancer in a patient by detecting at least 2 of the marker proteins selected from the markers of List 3, 4, 5, 6, 7, 8, 9, 10, 11, 12, 13, 14, 15, 16, 17, 18, 19, 20 or any combination thereof in a patient comprising the step of detecting antibodies binding said marker proteins, detecting said marker proteins or antigenic fragments thereof in a sample of the patient. Further provided is a method of diagnosing breast cancer or the risk of breast cancer in a patient by detecting at least 20%, preferably at least 30%, especially preferred at least 40%, at least 50%, at least 60%, at least 70%, at least 80% at least 90% or all, of the marker proteins selected from the markers of List 3, 4, 5, 6, 7, 8, 9, 10, 11, 12, 13, 14, 15, 16, 17, 18, 19, 20 or any combination thereof in a patient comprising the step of detecting antibodies binding said marker proteins, detecting said marker proteins or antigenic fragments thereof in a sample of the patient.

Also provided is a method of diagnosing breast cancer or the risk of breast cancer in a patient by detecting a marker protein selected from any one of List 1 in a patient comprising the step of detecting antibodies binding said marker protein, detecting said marker protein or antigenic fragments thereof in a sample of the patient. Of course, preferably more than one marker protein is detected. As noted with regards to the marker combinations of sets of lists 2 to 20, preferably at least 2, but also 3, 4, 5, 6, 7, 8, 9, 10, 11, 12, 13, 14, 15, 16, 17, 18, 19, 20, 21, 22, 23, 24, or 25 or more, of the inventive marker proteins can be detected. This relates to any one of the inventive sets of lists 1 to 20. Even more preferred at least 20%, at least 30%, at least 40%, at least 50%, at least 60%, at least 70%, at least 80%, at least 90%, at least 95% or all of the markers of any set of any of the lists 1 to 20 are used in a diagnostic set. Such parts of at least 2 markers or at least 20% markers (or more as indicated) are also referred to as subsets herein.

Such a marker combination of a particular list or any combination of marker selection thereof are referred to herein as diagnostic set. Such sets constitute a further aspect of the invention and kits are provided comprising diagnostic agents (such as binding moieties) to detect such markers. The entire disclosure herein relates to both the inventive kits (that can be used in the inventive methods) as well as the methods themselves, that make use of agents that can be comprised in the kit.

Preferred combinations are of markers that are particularly indicative for a specific distinction as given in table 1 below.

Preferred marker combinations are of 2, 3, or 4 lists selected from lists 3, 4, 5, and 6. These lists, as well as any combination are particularly effective for distinguishing indication 1, healthy conditions vs. benign tumor, and are preferably used therefore. Of course, not all of the markers are usually necessary since subsets also have sufficient diagnostic power. Preferably at least 2 markers or at least 20% of the markers (or any higher number as given above) of these lists or combined lists are used in the inventive methods.

Preferred marker combinations are of 2, 3, 4, 5, or 6 lists selected from lists 7, 8, 9, 10, 11, and 12. These lists, as well as any combination are particularly effective for distinguishing indication 2, healthy conditions vs. malign tumor and are preferably used therefore. Of course, not all of the markers are usually necessary since subsets also have sufficient diagnostic power. Preferably at least 2 markers or at least 20% of the markers (or any higher number as given above) of these lists or combined lists are used in the inventive methods.

Preferred marker combinations are of 2, 3, or 4 lists selected from lists 13, 14, 15, or 16. These lists, as well as any combination are particularly effective for distinguishing indication 3, benign tumor vs. malign tumor, and are preferably used therefore. Of course, not all of the markers are usually necessary since subsets also have sufficient diagnostic power. Preferably at least 2 markers or at least 20% of the markers (or any higher number as given above) of these lists or combined lists are used in the inventive methods.

Preferred marker combinations are of the 2 lists 17 and 18. These lists, as well as any combination are particularly effective for distinguishing indication 4, healthy conditions vs. hereditary breast cancer, and are preferably used therefore. Of course, not all of the markers are usually necessary since subsets also have sufficient diagnostic power. Preferably at least 2 markers or at least 20% of the markers (or any higher number as given above) of these lists or combined lists are used in the inventive methods.

A preferred marker combination is of the 2 lists 19 and 20. These lists, as well as any combination are particularly effective for distinguishing indication 5, malign tumor vs. hereditary breast cancer, and are preferably used therefore. Of course, not all of the markers are usually necessary since subsets also have sufficient diagnostic power. Preferably at least 2 markers or at least 20% of the markers (or any higher number as given above) of these lists or combined lists are used in the inventive methods.

In especially preferred embodiments, the combination is of lists 3, 4, 5, and 6, wherein the markers are selected from ARID5A, ATAD3A/ATAD3B, ATP5SL, BCL3, BSDC1, C12orf32, C1orf144, CCDC64, CDK11A/CDK11B, CDK5RAP2, CDT1, CELF5, CHKB, CTBP2, DDX19B, DDX24, E4F1, EFR3A, EIF3G, FAM171B, FAM208A, FIS1 (includes EG:288584), FKBP8, FURIN, GIPC1, GMFG, GTF2IRD1, HADH, HNRNPA1, HNRNPF, HNRNPUL1, HSPG2, HTT, ISG15, IST1 (includes EG:307833), KANSL2, KIF22, LCP1, MAGED2, MAPKAPK2, MED12, MED23, MEGF8, MGA, MKI67IP, MTUS1, MYH14, MYO9B, NARG2, NEDD4L, NFKBID, NOP58, NUMA1, OS9, P4HTM (includes EG:301008), PHACTR2, PIN1, PNPT1, PPM1G, PRPF31, PSMB8, RAB11FIP3, RAD21, RASGRP2, RRBP1, RSPH10B/RSPH10B2, SCAP, SERBP1, SETD4, SNRNP35, SORBS2, STX18, STXBP3, TAOK1, TNXB, TPM3, TUBA1B, TXN2, UBA1, USP12, VIM, VPS8 (includes EG:209018), VTI1B, ZC3H7A, ZC3H7B. These markers as well as the combined set of any one of at least 2 markers or at least 20% of said markers (or any higher number as indicated above) is particularly suitable for distinguishing healthy conditions vs. benign tumor and is preferably used for this diagnosis.

In especially preferred embodiments, the combination is of lists 7, 8, 9, 10, 11 and 12, wherein the markers are selected from AARS, ABAT, ACAT2, ACIN1, ACOT9, ACTN4, ACTR3, ADAMTS10, ADAMTS16, ADH5 (includes EG:100145871), AEBP1, AFMID, AGAP6 (includes others), AKR1C4, AKT1, AMBRA1, AMN1 (includes EG:196394), AMT, ANTXR1, ANXA6, AP2A1, APBB1, APBB1IP, APOH, ARCN1, ARHGAP1, ARHGAP44, ARHGEF11, ATIC, ATN1, ATP5SL, ATRX, ATXN1, AURKAIP1, BAZ1B, BCAM, BCL11A, BEX4, BIN1, BOD1L1, BRPF1, C12orf35, C12orf45, C8orf37, CACNB3, CAMSAP1, CCDC94, CCND1, CCT5, CD81, CD97, CDC25B, CDK13, CDT1, CENPF, CEP70, CHD3, CHMP1B, CINP, CISH, CLCN2, CLTC, CLU, CORO2B, CPE, CPNE1, CPNE6, CRAT, CSRNP1, CTBP2, CTSH, CYLD, DDX24, DDX39A, DENND1C, DFFA, DNAJC10, DOK2, E4F1, EEF1D, EEFSEC, EGR1, EHD4, EIF3G, EIF3J, EIF3L, EZR, FAM134A, FAM208A, FBRS, FBXL5, FBXO34, FBXO6, FCHO1, FGB, FIS1 (includes EG:288584), FLII, FLNA, FNTB, FOXL1, FTL, FUT8, GABARAPL2, GART, GBAS, GDAP1L1, GGA3, GLUL, GMPPB, GNAI2, GOLM1, GTF2IRD1, HARS, HIVEP1, HLA-A, HLA-B, HLA-C, HMGB2, HNRNPA1, HNRNPF, HNRNPUL1, HOOK2, HSP90AB1, HSPA1A/HSPA1B, HSPB1, HSPG2, HUWE1, IGF2R, IRF1 (includes EG:16362), IST1 (includes EG:307833), KANSL2, KAT7, KHDRBS1, KIF22, KLHDC3, KPNB1, KRT8, LAMC1, LCK, LRP1 (includes EG:16971), LRRC47, LRSAM1, LUC7L3, MAF1 (includes EG:315093), MAGED2, MAP3K3, MAP3K4, MAPK8IP2, MAPK8IP3, MCM3AP, MCM7, MED23, MGA, MGEA5, MINK1, MMP14, MTA2, MUC2 (includes EG:4583), MYH14, MYO9B, NAGLU, NAP1L1, NDC80, NEDD4L, NEFM, NISCH, NLE1, NOC2L, NONO, NOP58, NPM1, NRXN2, NT5C3L, NUMA1, NUP85 (includes EG:287830), OLFM2, OS9, OTUB1, PACS1, PCDH7, PCDH9, PDCD6IP, PDXK, PFAS, PITPNC1, PJA2, PLCE1, PLCG1, PLEKHH3, PLXNA1, PMS2, PNPT1, POGK, POGZ, POLR2H, PPIP5K1, PPP2R5D, PRDX1, PREPL, PRKD2, PRPF31, PRRC2C, PRRT1, PSMC4, PSMD13, PTK2B, PTPN23, PTPN7, PTPRA, PTPRF, PXN, QARS, RAB11FIP3, RAB5C, RABEPK, RAC2, RAD21, RAD9A, RAN, RARS, RBM25, RBM5, RGS2 (includes EG:19735), RNF146, RNF213, RPL10, RPLP0P2, RPS17/RPS17L, RPS18, RPS6KB2, RRBP1, RSPH10B/RSPH10B2, SAMD14, SBF1, SCAP, SCG5, SCO1, SDCBP, SEC13, SEPT1, SEPT6, SERBP1, SF3A3, SH3BGRL3, SH3BP1, SLC25A6, SLC7A5, SND1, SNRNP200, SNRNP35, SNX4, SNX5, SNX6, SORBS2, SPAG9, SPNS2, SPTBN1, SRA1, SRPR, STAB1, STAT1, STIP1, STMN4, STRN4, STX18, STXBP3, SUPT5H, TAF4B, TAOK1, TAX1BP1, THAP7, TIAF1, TMOD3, TMSB10/TMSB4X, TNFSF13, TPM2, TPM3, TPR, TRADD, TRIP12, TSC2, TSEN2, TTYH1, TXLNA, TXLNG2P, TYK2, U2AF1, UBA52, UBE2B, UBR4, UBR5, UQCRC1, URI1, USP12, VASP, VAV2, VIM, VPS37B, VPS72 (includes EG:100001285), WARS, WDR47, WDR6, WDTC1, ZC3H13, ZC3H7A, ZFP14, ZFP2, ZFYVE28, ZNF317, ZNF7, ZNFX1. These markers as well as the combined set of any one of at least 2 markers or at least 20% of said markers (or any higher number as indicated above) is particularly suitable for distinguishing healthy conditions vs. malign tumor and is preferably used for this diagnosis.

In especially preferred embodiments, the combination is of lists 13, 14, 15, and 16, wherein the markers are selected from A2M, ABCA11P, ABI1, ACTR1B, AIDA, AKAP9, AKR1C4, AMICA1, ANXA11, APEX1, ARAP1, ARCN1, ARF5, ARHGEF40, ARID4B, ARL8A, ATG16L1, ATP5D, ATP5SL, ATXN2, BCAM, BZRAP1, BZW2, C12orf45, C17orf56, C17orf70, C19orf25, C21orf2, CCDC149, CCL28, CCND1, CDC25B, CDC37, CECR5, CEP57, CHD1L, CIC, CNTD1, CNTROB, COL3A1, CPNE1, CSF1R, CSRNP1, CTSD, CUEDC2, CUL1, DBNL, DHX16, DHX9, DMPK, EGR1, EIF4G1, ERP29, EVPL, EZR, FAM192A, FIGNL1, FLNA, FLOT1, FOXP3, FUT8, FZR1, GABARAPL2, GLYR1, GNAS, GOLGA2, GOLGA7, GON4L, HK1, HMGB2, HMOX2, HNRNPA3, HNRNPUL1, HTATIP2, IGLC1, IRF1 (includes EG:16362), ITFG3, KCNJ14, KHDRBS1, KIAA1244, KLF5, LARP1, LCMT2, LOC440354, LPXN, LRRC47, LSP1 (includes EG:16985), MAGED2, MAPRE1, MCM5, MEGF8, METTL3, MICAL3, MORF4L1, MYO9A, NAP1L1, NCOA4, NEDD4L, NRG3, NXPH3, P4HB, PACS1, PARD3, PCGF2, PDZD4, PEF1, PIP5K1A, PKP3, PLCB3, POLI, POLR2H, POLR2L, POLR3E, POTEE/POTEF, PPM1G, PRKCD, PSMB3, PSME4, R3HDM4, RAB11FIP3, RASGRP1, RCBTB1, RCOR2, RGS2 (includes EG:19735), RIPK1, RNF34, RRBP1, RRM1, RSL24D1, SAFB2, SAMD14, SAP18, SFN, SIRT2, SLC7A5, SMAP2, SMCR8, SND1, SNX5, SPHK2, SPOCK2, SSBP2, STAT4, STAT6, STK10, SURF4, SURF6, TAF7, TALDO1, THAP7, THOP1, TNFRSF6B, TNRC18, TRAFD1, TRIM44, TSC2, TXLNG2P, TXNDC5, UNC45A, UTY, VPS72 (includes EG:100001285), WAC, WASH1/WASH5P, WDR33, WDR47, WDR73, XRCC1 (includes EG:22594), ZNF202, ZNF638, ZNF839. These markers as well as the combined set of any one of at least 2 markers or at least 20% of said markers (or any higher number as indicated above) is particularly suitable for distinguishing benign tumor vs. malign tumor and is preferably used for this diagnosis.

In especially preferred embodiments, the combination is of lists 17 and 18, wherein the markers are selected from ANXA6, ATIC, BAZ1B, BIN1, BIN3, CBX4, CCDC51, CCNL2, CDC37, CUL7, DCAF6, DSP, EHMT1, ENTPD6, ETFA, EXOSC10, EZH1, HEXDC, HMGN2, HSP90B1, IGHG1, IK, ILF3, IMMT, IMP4, KAT2A, KCMF1, KIFC3, LDHB (includes EG:3945), LOC284023, LRBA, LTBP3, MYCL1, NAPA, NEFL, NEU1, NUDT16L1, PACS1, PJA2, PLBD1, PLCB3, PLEKHA4, PLEKHO1, PLOD1, PLXNA3, PPID, PPIF, PPP1R9B, PRDX6, PSMB6, PTPRE, RCL1, RECQL4, RNF146, RPL37A, RPL7, SCAF1, SMARCC2, STX11, SWI5, TRO, TSC22D3, VIMP, WDR75. These markers as well as the combined set of any one of at least 2 markers or at least 20% of said markers (or any higher number as indicated above) is particularly suitable for distinguishing healthy conditions vs. hereditary breast cancer and are preferably used for this diagnosis.

In especially preferred embodiments, the combination is of lists 19 and 20, wherein the markers are selected from ACAT2, ACTN4, ADAMTS10, AGAP2, AIP, AKAP17A, AMBRA1, ANGPTL2, ANXA6, AP2M1, ARHGAP44, ARL4D, BCL11A, BIN3, BNIP1, BTBD7, C3orf19, CANX, CDT1, CELSR1, CORO2B, CTSH, D2HGDH, DFFA, EHMT1, EIF3G, EIF4B, FAM69B, FLII, GLUL, HIST3H2A, HLA-C, HLA-C, HNRNPA0, HNRNPH1, HNRNPK, HNRPDL, HSPA1A/HSPA1B, HSPA9, IGF2R, IK, IK, INPPL1, KCNK5, KIFC3, LCK, LGALS3, LONP1, MAP3K4, MCM3AP, METTL17, MSLN, MTA2, NAPA, NEFM, NISCH, NOC2L, NRBP2, NUP93, PA2G4, PCDH9, PDE2A, PGD, PGRMC1, PLEKHH3, PLOD1, PLXNA3, POLR2B, PRDX6, PTPRA, PTPRE, PTPRF, PUF60, PUF60, PXN, RBM10, RNF213, RNF219, RNF34, RPLP0P2, RSPRY1, SAT2, SBF1, SEPT1, SLC7A5, SLU7 (includes EG:10569), SPTAN1, SSRP1, ST3GAL3, STOML2, TBC1D9B, TF, TRIP12, TSEN2, TTC3, ZNF132, ZNF300, ZNF431, ZNF7, ZSWIM4. These markers as well as the combined set of any one of at least 2 markers or at least 20% of said markers (or any higher number as indicated above) is particularly suitable for distinguishing malign tumor vs. hereditary breast cancer and are preferably used for this diagnosis.

Some markers are more preferred than others. Especially preferred markers are those which are represented at least 5, at least 6, at least 7, at least 8, at least 9, at least 10, at least 11, at least 12 times in any one of lists 3 to 20. These markers are preferably used in any one of the inventive methods or sets.

Less preferred markers are selected from ASB-9, SERAC1, RELT, P16, P53, C-MYC, PPIA, PRDX2, FKBP52, MUC1, HPS60, HER2, NY-ESO-1, BRCA2, HSP60, PHB, TUBB, IMP1, P62, KOC, CCNB1.

Preferably none of these markers is used in the inventive methods or present in one of the inventive set.

The present invention also relates to a method of selecting such at least 2 markers (or more as given above) or at least 20% of the markers (or more as given above) of any one of the inventive sets with high specificity. Such a method includes comparisons of signal data for the inventive markers of any one of the inventive markers sets, especially as listed in lists 1 to 20, with said signal data being obtained from control samples of known conditions or indications and further statistically comparing said signal data with said conditions thereby obtaining a significant pattern of signal data capable of distinguishing the conditions of the known control samples.

In particular, the controls may comprise one or more cancerous control (preferably at least 5, or at least 10 cancerous controls) and a healthy control (preferably at least 5, or at least 10 healthy controls). Preferably 2 different indications are selected that shall be distinguished. In preferred embodiments, the control comprises samples for the indications selected from indications 1), 2), 3), 4), and 5) as mentioned above.

The controls can be used to obtain a marker dependent signal pattern as indication classifier. Such a signal pattern can be obtained by routine statistical methods, such as binary tree methods. Common statistical methods calculate a (optionally multi-dimensional) vector within the multitude of control data signal values as diagnostically significant distinguishing parameter that can be used to distinguish one or more indications from other one or more indications. The step usually comprises the step of "training" a computer software with said control data. Such pre-obtained training data or signal data can be provided on a computer-readable medium to a practitioner who performs the inventive diagnosis.

Preferably, the method comprises optimizing the selection process, e.g. by selecting alternative or additional markers and repeating said comparison with the controls signals, until a specificity and/or sensitivity of at least 75% is obtained, preferably of at least 80%, at least 85%, at least 90%, at least 95%.

"Marker" or "marker proteins" are diagnostic indicators found in a patient and are detected, directly or indirectly by the inventive methods. Indirect detection is preferred. In particular, all of the inventive markers have been shown to cause the production of (auto)antigens in cancer patients or patients with a risk of developing cancer. The easiest way to detect these markers is thus to detect these (auto)antibodies in a blood or serum sample from the patient. Such antibodies can be detected by binding to their respective antigen in an assay. Such antigens are in particular the marker proteins themselves or antigenic fragments thereof. Suitable methods exist in the art to specifically detect such antibody-antigen reactions and can be used according to the invention. Preferably the entire antibody content of the sample is normalized (e.g. diluted to a preset concentration) and applied to the antigens. Preferably the IgG, IgM, IgD, IgA or IgE antibody fraction, is exclusively used. Preferred antibodies are IgG. Preferably the subject is a human.

Binding events can be detected as known in the art, e.g. by using labeled secondary antibodies. Such labels can be enzymatic, fluorescent, radioactive or a nucleic acid sequence tag. Such labels can also be provided on the binding means, e.g. the antigens as described in the previous paragraph. Nucleic acid sequence tags are especially preferred labels since they can be used as sequence code that not only leads to quantitative information but also to a qualitative identification of the detection means (e.g. antibody with certain specificity). Nucleic acid sequence tags can be used in known methods such as Immuno-PCR. In multiplex assays, usually qualitative information is tied to a specific location, e.g. spot on a microarray. With qualitative information provided in the label, it is not necessary to use such localized immunoassays. It is possible to perform the binding reaction of the analyte and the detection means, e.g. the serum antibody and the labeled antigen, independent of any solid supports in solution and obtain the sequence information of the detection means bound to its analyte. A binding reaction allows amplification of the nucleic acid label in a detection reaction, followed by determination of the nucleic acid sequence determination. With said determined sequence the type of detection means can be determined and hence the marker (analyte, e.g. serum antibody with tumor associated antigen specificity).

In preferred embodiments of the invention the step of detecting antibodies binding said marker proteins, detecting said marker proteins or antigenic fragments thereof comprises comparing said detection signal with detection signals of a healthy control and comparing said detection signals, wherein an increase in the detection signal indicates breast cancer or said risk of breast cancer.

In preferred embodiments of the invention the step of detecting antibodies binding said marker proteins, detecting said marker proteins or antigenic fragments thereof comprises comparing said detection signal with detection signals of a cancerous control. In preferred embodiments, the control comprises the indications that are intended to be distinguished, such as indications 1), 2), 3), 4), and 5)as mentioned above. In particular preferred, especially in cases of using more marker sets of 2 or more markers as mentioned above, a statistical analysis of the control is performed, wherein the controls are used to obtain a marker dependent signal pattern as indication classifier and the marker dependent signals of the sample to be analysed is compared with and/or fitted onto said pattern thereby obtaining information of the diagnosed condition or indication. Such a signal pattern can be obtained by routine statistical methods, such as binary tree methods. Common statistical methods calculate a (optionally multi-dimensional) vector within the multitude of control data signal values as diagnostically significant distinguishing parameter that can be used to distinguish one or more indications from other one or more indications. Such statistical analysis is usually dependent on the used analytical platform that was used to obtain the signal data, given that signal data may vary from platform to platform. Such platforms are e.g. different microarray or solution based setups (with different labels or analytes - such as antigen fragments - for a particular marker). Thus the statistical method can be used to calibrate each platform to obtain diagnostic information with high sensitivity and specificity. The step usually comprises the step of "training" a computer software with said control data. Alternatively, pre-obtained training data can be used. Such pre-obtained training data or signal data can be provided on a computer-readable medium to a practitioner.

In further embodiments a detection signal from the sample of a patient in amplitude of at least 60%, preferably at least 80%, of the cancerous control indicates breast cancer.

Usually not all of the inventive markers or detection agents may lead to a signal. Nevertheless only a fraction of the signals is suitable to arrive at a diagnostic decision. In preferred embodiments of the invention a detection signal in at least 60%, preferably at least 70%, least 75%, at least 85%, or in particular preferred at least 95%, even more preferred all, of the used markers indicates breast cancer.

The present diagnostic methods further provide necessary therapeutic information to decide on a surgical intervention. Therefore the present invention also provides a method of treating a patient comprising breast cancer, comprising detecting cancer according to any aspect or embodiment of the invention and removing said breast cancer. "Stratification or therapy control" for the purposes of this invention means that the method according to the invention renders possible decisions for the treatment and therapy of the patient, whether it is the hospitalization of the patient, the use, effect and/or dosage of one or more drugs, a therapeutic measure or the monitoring of a course of the disease and the course of therapy or etiology or classification of a disease, e.g., into a new or existing subtype or the differentiation of diseases and the patients thereof. In a further embodiment of the invention, the term "stratification" covers in particular the risk stratification with the prognosis of an outcome of a negative health event.

One skilled in the art is familiar with expression libraries, they can be produced according to standard works, such as Sambrook et al, "Molecular Cloning, A laboratory handbook, 2nd edition (1989), CSH press, Cold Spring Harbor, N.Y. Expression libraries are also preferred which are tissue-specific (e.g., human tissue, in particular human organs). Members of such libraries can be used as inventive antigen for use as detection agent to bind analyte antibodies. Furthermore included according to the invention are expression libraries that can be obtained by exon-trapping. A synonym for expression library is expression bank. Also preferred are protein biochips or corresponding expression libraries that do not exhibit any redundancy (so-called: Uniclone(R) library) and that may be produced, for example, according to the teachings of WO 99/57311 and WO 99/57312. These preferred Uniclone libraries have a high portion of nondefective fully expressed proteins of a cDNA expression library. Within the context of this invention, the antigens can be obtained from organisms that can also be, but need not be limited to, transformed bacteria, recombinant phages, or transformed cells from mammals, insects, fungi, yeasts, or plants. The marker antigens can be fixed, spotted, or immobilized on a solid support. Alternatively, is also possible to perform an assay in solution, such as an Immuno-PCR assay.

In a further aspect, the present invention provides a kit of diagnostic agents suitable to detect any marker or marker combination as described above, preferably wherein said diagnostic agents comprise marker proteins or antigenic fragments thereof suitable to bind antibodies in a sample, especially preferred wherein said diagnostic agents are immobilized on a solid support or in solution, especially when said markers are each labelled with a unique label, such as a unique nucleic acid sequence tag. The inventive kit may further comprise detection agents, such as secondary antibodies, in particular anti-human antibodies, and optionally also buffers and dilution reagents. The invention therefore likewise relates to the object of providing a diagnostic device or an assay, in particular a protein biochip, ELISA or Immuno-PCR assay, which permits a diagnosis or examination for breast carcinoma.

Additionally, the marker proteins (as binding moieties for antibody detection) can be present in the respective form of a fusion protein, which contains, for example, at least one affinity epitope or tag. The tag may be one such as contains c-myc, his tag, arg tag, FLAG, alkaline phosphatase, VS tag, T7 tag or strep tag, HAT tag, NusA, S tag, SBP tag, thioredoxin, DsbA, a fusion protein, preferably a cellulose-binding domain, green fluorescent protein, maltose-binding protein, calmodulin-binding protein, glutathione S-transferase, or lacZ, a nanoparticle or a nucleic acid sequence tag. Such a nucleic acid sequence can be e.g. DNA or RNA, preferably DNA.

In all of the embodiments, the term "solid support" covers embodiments such as a filter, a membrane, a magnetic or fluoro-phore-labeled bead, a silica wafer, glass, metal, ceramics, plastics, a chip, a target for mass spectrometry, or a matrix. However, a filter is preferred according to the invention.

As a filter, furthermore PVDF, nitrocellulose, or nylon is preferred (e.g., Immobilon P Millipore, Protran Whatman, Hybond N+ Amersham).

In another preferred embodiment of the arrangement according to the invention, the arrangement corresponds to a grid with the dimensions of a microtiter plate (8-12 wells strips, 96 wells, 384 wells, or more), a silica wafer, a chip, a target for mass spectrometry, or a matrix.

Preferably the inventive kit also comprises non-diagnostic control proteins, which can be used for signal normalization. These control proteins bind to moieties, e.g. proteins or antibodies, in the sample of a diseased patient same as in a healthy control. In addition to the inventive marker proteins any number, but preferably at least 2 controls can be used in the method or in the kit.

Preferably the inventive kit is limited to a particular size. According to these embodiments of the invention the kit comprises at most 3000 diagnostic agents, preferably at most 2500 diagnostic agents, at most 2000 diagnostic agents, at most 1500 diagnostic agents, at most 1200 diagnostic agents, at most 1000 diagnostic agents, at most 800 diagnostic agents, at most 500 diagnostic agents, at most 300 diagnostic agents, at most 200 diagnostic agents, at most 100 diagnostic agents, such as marker proteins or antigenic fragments thereof.

In especially preferred embodiments of the invention the kit further comprises a computer-readable medium or a computer program product, such as a computer readable memory devices like a flash storage, CD-, DVD- or BR-disc or a hard drive, comprising signal data for the control samples with known conditions selected from cancer and/or of healthy controls, and/or calibration or training data for analysing said markers provided in the kit for diagnosing breast cancer or distinguishing conditions or indications selected from healthy conditions and cancer. Especially preferred are the indications 1), 2), 3), 4), and 5) mentioned above.

The kit may also comprise normalization standards, that result in a signal independent of a healthy condition and cancerous condition. Such normalization standards can be used to obtain background signals. Such standards may be specific for ubiquitous antibodies found in a human, such as antibodies against common bacteria such as *E*. *coli.* Preferably the normalization standards include positive and negative (leading to no specific signal) normalization standards.

The present invention is further illustrated by the following figures and examples, without being limited to these embodiments of the invention.

### Figures:

Fig. **1****:** Example of a scanned 16k protein-microarray. In the detail right side, subarray 6 is depicted.
Fig. 2: Subsets of classifier markers form the panels defined by the 50 classifier markers for distinguishing malign tumor vs.
controls were selected and randomly chosen subsets of 2-49 markers (x-axis) were selected 1000-times. The classification success obtained by that subset using the nearest centroid classifier algorithm is shown on the y-axis.
Fig. 3: The 50 classifier markers for distinguishing malign tumor vs. benign tumors were selected and randomly chosen subsets of 2-49 markers (x-axis) were selected 1000-times. The classification success obtained by that subset using the nearest centroid classifier algorithm is shown on the y-axis.

### Examples:

### Example 1: Patient samples

Biomarker screening has been performed with purified IgG samples from a test set of serum samples derived from 95 individuals with confirmed breast carcinoma, comprising 78 patients with sporadic breast cancer, 17 patients with hereditary breast cancer (with BRCA1 or BRCA2 mutation), 46 patients with benign breast tumor and 62 healthy controls (n=203).

### Example 2: Immunoglobuline (IgG) purification from the serum or serum samples

The patient serum or serum samples were stored at -80 °C before they were put on ice to thaw them for IgG purification using Melon Gel 96-well Spin Plate according the manufacturer's instructions (Pierce). In short, 10µl of thawed sample was diluted in 90 µl of the equilibrated purification buffer on ice, then transferred onto Melon Gel support and incubated on a plate shaker at 500 rpm for 5 minutes. Centrifugation at 1,000 x g for 2 minutes was done to collect the purified IgG into the collection plate.

Protein concentrations of the collected IgG samples were measured by absorbance measures at 280nm using an Epoch Micro-Volume Spectrophotometer System (Biotec,USA). IgG-concentrations of all samples were concentration-adjusted and 0.6 mg/ml of samples were diluted 1:1 in PBS2x buffer with TritonX 0.2% and 6% skim milk powder for microarray analyses.

### Example 3: Microarray design

A protein-chip named "16k protein chip" from 15284 human cDNA expression clones derived from the Unipex cDNA expression library plus technical controls was generated. Using this 16k protein chip candidate markers were used to identify autoantibody profiles suitable for unequivocal distinction of healthy conditions and breast cancer.

Protein-microarray generation and processing was using the Unipex cDNA expression library for recombinant protein expression in E.coli. His-tagged recombinant proteins were purified using Ni-metal chelate chromatography and proteins were spotted in duplicates for generation of the microarray using ARChipEpoxy slides.

### Example 4: Preparation, processing and analyses of protein microarrays

The microarray with printed duplicates of the protein marker candidates was blocked with DIG Easy Hyb (Roche) in a stirred glass tank for 30 minutes. Blocked slides were washed 3x for 5 minutes with fresh PBSTritonX 0.1% washing buffer with agitation. The slides were rinsed in distilled water for 15 seconds to complete the washing step and remove leftovers from the washing buffer. Arrays were spun dry at 900rpm for 2 minutes. Microarrays were processed using the Agilent Microarray Hybridisation Chambers (Agilent) and Agilent's gasket slides filled with 490 µl of the prepared sample mixture and processed in a hybridization oven for 4 h at RT with a rotation speed of 12. During this hybridization time the samples were kept under permanent rotating conditions to assure a homolog dispensation.

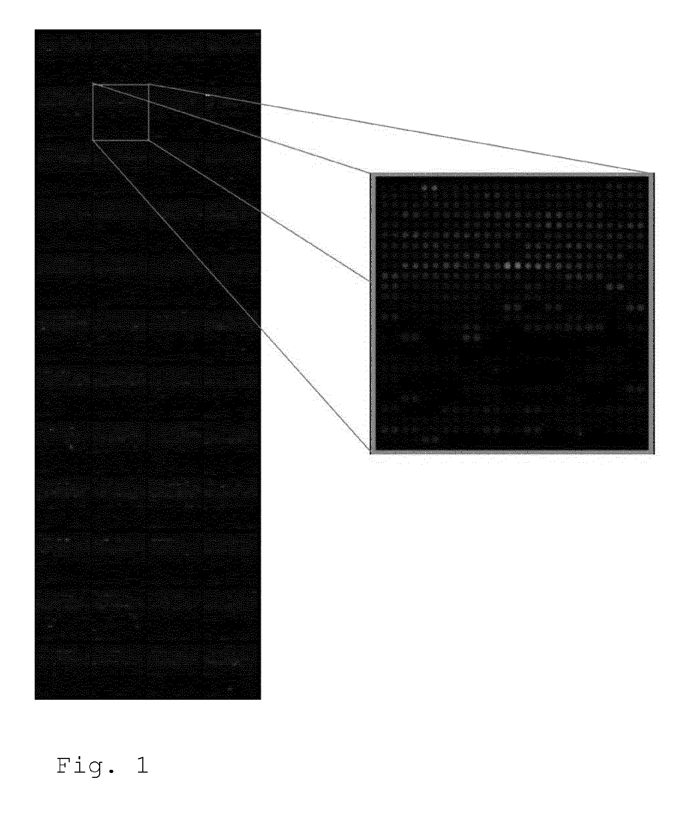

After the hybridization was done, the microarray slides were washed 3x with the PBSTritonX 0.1% washing buffer in the glass tank with agitation for 5 minutes and rinsed in distilled water for about 15 seconds. Then, slides were dried by centrifugation at 900 rpm for 2 minutes. IgG bound onto the features of the protein-microarrays were detected by incubation with cy5 conjugated Alexa Fluor® 647 Goat Anti-Human IgG (H+L) (Invitrogen, Lofer, Austria), diluted in 1:10,000 in PBSTritonX 0.1% and 3% skim milk powder using rotating conditions for 1h, with a final washing step as outlined above. Microarrays were then scanned and fluorescent data extracted from images (Fig. 1) using the GenePixPro 6.0 software (AXON).

### Example 5: Data analysis

Data were quantile normalised; data analyses was conducted using BRB array tools (web at linus.nci.nih.gov/BRB-ArrayTools.html).

For identification of tumor marker profiles and classifier markers, class prediction analyses applying leave-one-out cross-validation was used. Classifiers were built for distinguishing each of the five classes of samples denoted "Mal." (Malign tumors) patients, "Ben." patients harboring benign tumors, "Her." patients harboring hereditary breast cancer, and "Contr" individuals with no carcinoma or tumor. In addition different combinations of classes were also built as listed in the table below (Tab. 1) and again class prediction analysis was conducted for differentiation of these different combinations.

**Table 1: Breast tumor marker Classifiers defined for separation of different indications (Contr.: for age statistically balanced controls; Mal.: Malign tumor; Ben.: Benign tumor ; Her.: hereditary breast cancer;).**

| Contrast analysed | examples with quantile normalisation |
|---|---|
| 1) Contr. vs Ben. | 7.1 - 7.4 |
| 2) Contr. vs Mal. | 7.5 - 7.10 |
| 3) Ben. vs Mal. | 7.11 - 7.14 |
| 4) Contr. vs Her. | 7.15, 7.16 |
| 5) Mal. vs Her. | 7.17, 7.18 |

### Example 6: Results Summary

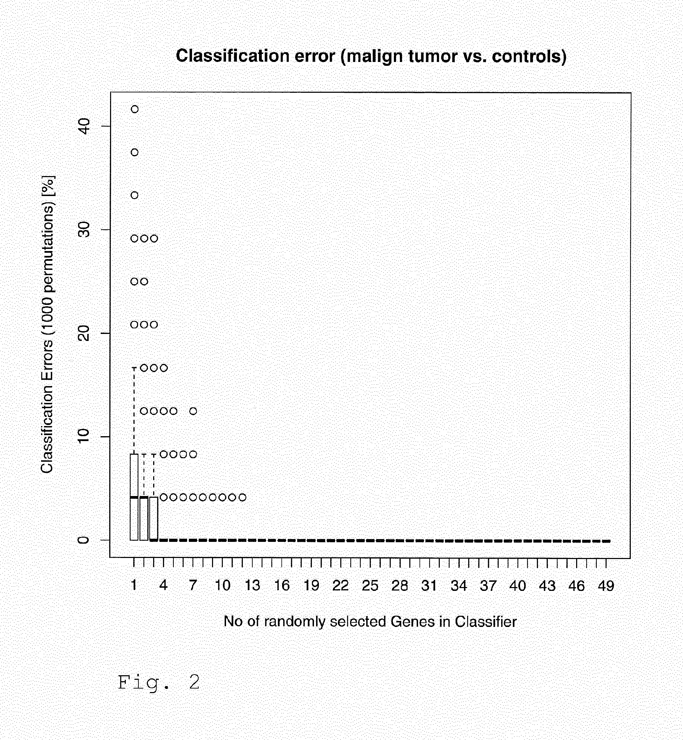

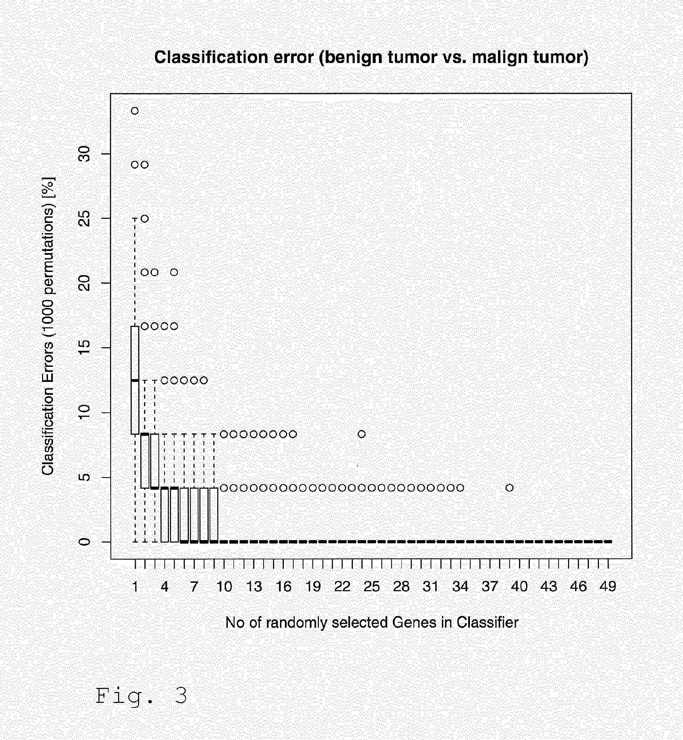

For distinguishing 1) healthy conditions vs. benign tumor, 2) healthy conditions vs. malign tumor, 3) benign tumor vs. malign tumor, 4) healthy conditions vs. hereditary breast cancer, and 5) malign tumor vs. hereditary breast cancer, 63 genes were present in at least 5 classifier lists. The classification success with respect to different contrasts (differentiation of different patient classes and combinations thereof) and presence of 63 preferred List 2 markers is given in Table 2. As shown, the number of markers out of the 63 selected. It was also shown that using only isolated or only 2 markers from the present classifier sets enables correct classification of >55% (Example 8, Fig. 2 & 3). Therefore the marker-lists, subsets and single markers (antigens; proteins; peptides) are of particular diagnostic values.

In addition it has already been shown that peptides deduced from proteins or seroreactive antigens can be used for diagnostics and in the published setting even improve classification success (Syed 2012; Journal of Molecular Biochemistry; Vol 1, No 2, www.jmolbiochem.com/index.php/JmolBiochem/article/view/54).

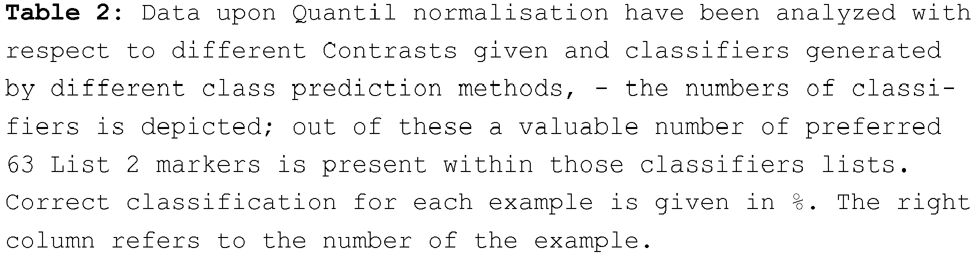

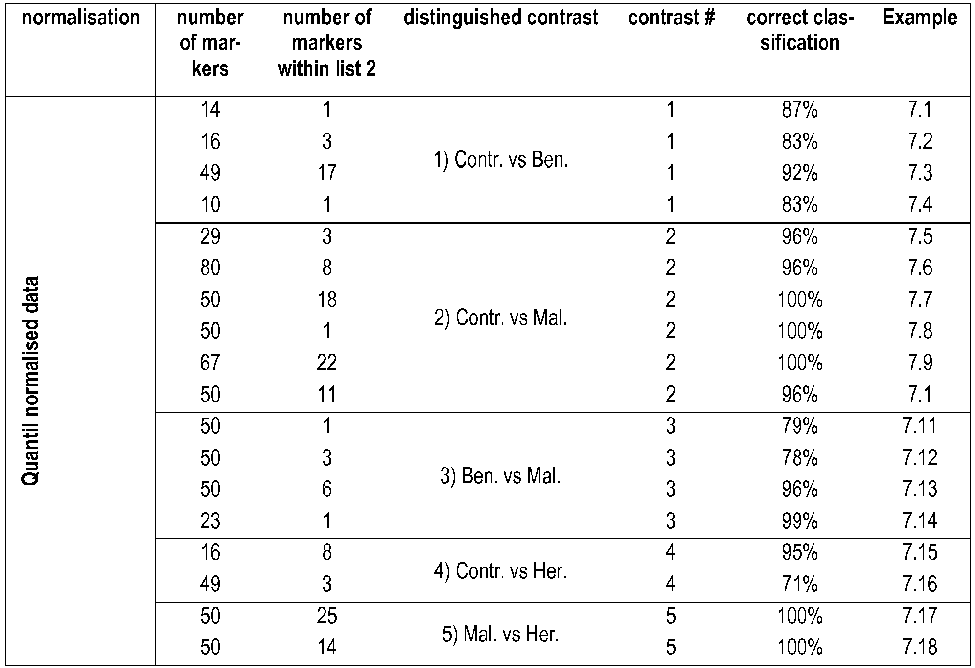

A classifier list has been elucidated for the "contrasts" listed in Table 1, - upon quantile normalization (QNORM)). Classifier markers (n=591) were identified according to List 1.

### Example 7: Detailed Results

### Quantile-normalized data

### Example 7.1: "Healthy conditions vs. benign tumor" (using just runs 1) - p<0.001 > 87% CCP, DLDA

The following markers were identified according to this example:

**List 3:** FURIN, GTF2IRD1, LCP1, MAGED2, MEGF8, NARG2, PIN1, PRPF31, RASGRP2, SNRNP35, STX18, TNXB, TXN2, VTI1B. Features "significantly different between the classes at 0.001 significance level" were used for class prediction of "healthy conditions vs. benign tumor" samples including benign tumor cases versus their statistically age-balanced Contr samples (contrast 1) using 23 samples (11 control, 12 benign tumor samples). Using features significantly different between the classes at 0.001 significance level, for those 14 features the Compound Covariate Predictor (CCP) and the Diagonal Linear Discriminant Analysis (DLDA) predictor enabled correct classification of 87% of the tested samples.

Feature subsetting and prediction was performed repeatedly for each of the K-fold (K=23) cross-validations of this subsetting method. By that means the rate of correct classification was calculated.

### Performance of classifiers during cross-validation.

| | **CompoundCovariatePredictor** | **Diagonal Line-arDiscriminan-tAnalysis** | **1-Nearest-Neighbor** | **3-Nearest-Neighbors** | **Nearest-Centroid** | **SupportVec-torMachines** | **BayesianCompound-CovariatePredictor** |
|---|---|---|---|---|---|---|---|
| Mean percentof correctclas-sification: | 87 | 87 | 74 | 74 | 83 | 65 | 89 |

### Performance of the Compound Covariate Predictor Classifier:

| **Class** | **Sensitivity** | **Specificity** | **PPV** | **NPV** |
|---|---|---|---|---|
| **Ben** | 0.833 | 0.909 | 0.909 | 0.833 |
| **Con** | 0.909 | 0.833 | 0.833 | 0.909 |

### Performance of the Diagonal Linear Discriminant Analysis Classifier:

| **Class** | **Sensitivity** | **Specificity** | **PPV** | **NPV** |
|---|---|---|---|---|
| **Ben** | 0.833 | 0.909 | 0.909 | 0.833 |
| **Con** | 0.909 | 0.833 | 0.833 | 0.909 |

### Example 7.2: "Healthy conditions vs. benign tumor" (using just run 2) - p<0.001 > 83% CCP, DLDA, 1NN, 3NN, NC

The following markers were identified according to this example: **List 4:** CTBP2, E4F1, FIS1 (includes EG:288584), HNRNPF, HNRNPUL1, MYO9B, NEDD4L, NOP58, OS9, PHACTR2, RSPH10B/RSPH10B2, SCAP, SERBP1, SORBS2, STXBP3, USP12.

Features "significantly different between the classes at 0.001 significance level" were used for class prediction of "healthy conditions vs. benign tumor" samples including benign tumor cases versus their statistically age-balanced Contr samples (contrast 1) using 24 samples (12 control and 12 benign tumor samples). Using features significantly different between the classes at 0.001 significance level, for those 16 features the Compound Covariate Predictor (CCP), the Diagonal Linear Discriminant Analysis (DLDA), 1-Nearest-Neighbour (1NN), 3-Nearest-Neighbours (3NN), and the Nearest Centroid (NC) predictor enabled correct classification of 83% of the tested samples. Feature subsetting and prediction was performed repeatedly for each of the K-fold (K=24) cross-validations of this subsetting method. By that means the rate of correct classification was calculated.

### Performance of classifiers during cross-validation.

| | **CompoundCova-riatePredictor** | **Diagonal Line-arDiscriminan-tAnalysis** | **1-Nearest-Neighbor** | **3-Nearest-Neighbors** | **Nearest-Centroid** | **SupportVec-torMachines** | **BayesianCompound-CovariatePredictor** |
|---|---|---|---|---|---|---|---|
| Mean percentof correctclas-sification: | 83 | 83 | 83 | 83 | 83 | 79 | 83 |

### Performance of the Compound Covariate Predictor Classifier:

| **Class** | **Sensitivity** | **Specificity** | **PPV** | **NPV** |
|---|---|---|---|---|
| **Ben** | 0.833 | 0.833 | 0.833 | 0.833 |
| **Contr** | 0.833 | 0.833 | 0.833 | 0.833 |

### Performance of the Diagonal Linear Discriminant Analysis Classifier:

| **Class** | **Sensitivity** | **Specificity** | **PPV** | **NPV** |
|---|---|---|---|---|
| **Ben** | 0.833 | 0.833 | 0.833 | 0.833 |
| **Contr** | 0.833 | 0.833 | 0.833 | 0.833 |

### Performance of the 1-Nearest Neighbor Classifier:

| **Class** | **Sensitivity** | **Specificity** | **PPV** | **NPV** |
|---|---|---|---|---|
| **Ben** | 0.833 | 0.833 | 0.833 | 0.833 |
| **Contr** | 0.833 | 0.833 | 0.833 | 0.833 |

### Performance of the 3-Nearest Neighbors Classifier:

| **Class** | **Sensitivity** | **Specificity** | **PPV** | **NPV** |
|---|---|---|---|---|
| **Ben** | 0.833 | 0.833 | 0.833 | 0.833 |
| **Contr** | 0.833 | 0.833 | 0.833 | 0.833 |

### Performance of the Nearest Centroid Classifier:

| **Class** | **Sensitivity** | **Specificity** | **PPV** | **NPV** |
|---|---|---|---|---|
| **Ben** | 0.833 | 0.833 | 0.833 | 0.833 |
| **Contr** | 0.833 | 0.833 | 0.833 | 0.833 |

### Example 7.3: "Healthy conditions vs. benign tumor" (using just run 3) - 25GP > 92% 1NN, SVM

The following markers were identified according to this example: **List 5:** ARID5A, ATAD3A/ATAD3B, ATP5SL, BCL3, BSDC1, C12orf32, C1orf144, CCDC64, CDK11A/CDK11B, CDK5RAP2, CDT1, CELF5, DDX19B, DDX24, EIF3G, FAM171B, FAM208A, FKBP8, GIPC1, GMFG, HADH, HNRNPA1, HNRNPUL1, HSPG2, HTT, ISG15, IST1 (includes EG:307833), KANSL2, KIF22, MAGED2, MED12, MED23, MGA, MTUS1, MYH14, NFKBID, NUMA1, PNPT1, PPM1G, RAB11FIP3, RAD21, RRBP1, SETD4, TAOK1, TPM3, UBA1, VIM, VPS8 (includes EG:209018), ZC3H7B.

The "25 greedy pairs" (25GP) strategy was used for class prediction of "healthy conditions vs. benign tumor" samples including benign tumor cases versus their statistically age-balanced Contr samples (contrast 1) using 24 samples (12 control and 12 benign tumor samples). Using the "25 greedy pairs" the 1-Nearest-Neighbor (1NN) predictor and the Support Vector Machine (SVM) predictor enabled correct classification of 92% of the tested samples.

Feature subsetting and prediction was performed repeatedly for each of the K-fold (K=24) cross-validations of this subsetting method. By that means the rate of correct classification was calculated.

### Performance of classifiers during cross-validation.

| | **CompoundCova-riatePredictor** | **Diagonal LinearDiscriminantAnalysis** | **1-Nearest-Neighbor** | **3-Nearest-Neighbors** | **Nearest-Centroid** | **SupportVec-torMachines** | **BayesianCompound-CovariatePredictor** |
|---|---|---|---|---|---|---|---|
| Mean percentof correctclas-sification: | 88 | 88 | 92 | 88 | 88 | 92 | 88 |

### Performance of the 1-Nearest Neighbor Classifier:

| **Class** | **Sensitivity** | **Specificity** | **PPV** | **NPV** |
|---|---|---|---|---|
| **Ben** | 0.917 | 0.917 | 0.917 | 0.917 |
| **Ctrl** | 0.917 | 0.917 | 0.917 | 0.917 |

### Performance of the Support Vector Machine Classifier:

| **Class** | **Sensitivity** | **Specificity** | **PPV** | **NPV** |
|---|---|---|---|---|
| **Ben** | 0.917 | 0.917 | 0.917 | 0.917 |
| **Ctrl** | 0.917 | 0.917 | 0.917 | 0.917 |

### Example 7.4: "Healthy conditions vs. benign tumor" (using just run 4) - p<0.001 > 83% SVM

The following markers were identified according to this example:

**List 6:** CHKB, EFR3A, HSPG2, KANSL2, MAPKAPK2, MKI67IP, P4HTM (includes EG:301008), PSMB8, TUBA1B, ZC3H7A. Features "significantly different between the classes at 0.001 significance level" were used for class prediction of "healthy conditions vs. benign tumor" samples including benign tumor cases versus their statistically age-balanced Contr samples (contrast 1) using 18 samples (11 control and 7 benign tumor samples). Using features significantly different between the classes at 0.001 significance level, for those 10 features the Support Vector Machine (SVM) predictor enabled correct classification of 83% of the tested samples.

Feature subsetting and prediction was performed repeatedly for each of the K-fold (K=18) cross-validations of this subsetting method. By that means the rate of correct classification was calculated.

### Performance of classifiers during cross-validation.

| | **CompoundCova-riatePredictor** | **Diagonal Line-arDiscriminan-tAnalysis** | **1-Nearest-Neighbor** | **3-Nearest-Neighbors** | **Nearest-Centroid** | **SupportVec-torMachines** | **BayesianCompound-CovariatePredictor** |
|---|---|---|---|---|---|---|---|
| Mean percentof correctclas-sification: | 72 | 72 | 67 | 72 | 72 | 83 | 76 |

### Performance of the Support Vector Machine Classifier:

| **Class** | **Sensitivity** | **Specificity** | **PPV** | **NPV** |
|---|---|---|---|---|
| **Ben** | 0.857 | 0.818 | 0.75 | 0.9 |
| **Ctrl** | 0.818 | 0.857 | 0.9 | 0.75 |

### Example 7.5: "healthy conditions vs. malign tumor" (using just run 1) - p<0.001 > 96% SVM The following markers were identified according to this example:

**List 7:** ABAT, ACOT9, ACTN4, APBB1, BCAM, BIN1, CCT5, CPE, DNAJC10, GTF2IRD1, HMGB2, IRF1 (includes EG:16362), IST1 (includes EG:307833), KRT8, NRXN2, PITPNC1, PPP2R5D, PRPF31, PRRT1, SAMD14, SDCBP, SNRNP200, SNRNP35, SPAG9, WDR47, ZFP14, ZFYVE28, ZNF317, ZNFX1.

Features "significantly different between the classes at 0.001 significance level" were used for class prediction of "healthy conditions vs. malign tumor" samples including malignant tumor cases versus their statistically age-balanced Contr samples (contrast 2) using 23 samples (11 control and 12 malignant tumor samples). Using features significantly different between the classes at 0.001 significance level, for those 29 features the Support Vector Machines (SVM) enabled correct classification of 96% of the tested samples. Prior to feature subsetting features with less than 20 % of expression data having least a 1.5 -fold change in either direction from gene's median value, a percentile of the log-ratio variation in less than 75, and the 50th Percentile of intensities with less than a value of 500 got filtered out. Feature subsetting and prediction was performed repeatedly for each of the K-fold (K=23) cross-validations of this subsetting method. By that means the rate of correct classification was calculated.

### Performance of classifiers during cross-validation.

| | **Compound-CovariatePredic-tor** | **Diagonal Lin-earDiscrimi-nantAnalysis** | **1-Nearest-Neighbor** | **3-Nearest-Neighbors** | **Near-estCen-troid** | **SupportVec-torMachines** | **BayesianCompound-CovariatePredictor** |
|---|---|---|---|---|---|---|---|
| Mean percentof correctclas-sification: | 91 | 91 | 91 | 91 | 91 | 96 | 91 |

### Performance of the Support Vector Machine Classifier:

| **Class** | **Sensitivity** | **Specificity** | **PPV** | **NPV** |
|---|---|---|---|---|
| **Healthy** | 1 | 0.917 | 0.917 | 1 |
| **Mal** | 0.917 | 1 | 1 | 0.917 |

### Example 7.6: "healthy conditions vs. malignant tumor" (using just run 2) - p<0.001 > 96% CCP, DLDA, 1NN, 3NN, NC, SVM

The following markers were identified according to this example: **List 8:** ACTN4, AEBP1, ANXA6, APOH, BOD1L1, BRPF1, C12orf45, CCDC94, CDK13, CENPF, CEP70, CHD3, CLTC, CSRNP1, E4F1, EEFSEC, EIF3G, FAM134A, FBXO34, FCHO1, FIS1 (includes EG:288584), FLII, FNTB, FOXL1, GABARAPL2, GDAP1L1, HLA-C, HMGB2, HNRNPF, HNRNPUL1, HSP90AB1, IRF1 (includes EG:16362), KAT7, KHDRBS1, KLHDC3, LRP1 (includes EG:16971), MAF1 (includes EG:315093), MAPK8IP2, MYO9B, NEDD4L, NOP58, NPM1, NT5C3L, OS9, PFAS, PLCG1, POGK, POGZ, PPIP5K1, PRDX1, PREPL, PRKD2, PRRC2C, PTPN23, PXN, QARS, RAB5C, RBM25, RBM5, RSPH10B/RSPH10B2, SCAP, SERBP1, SLC7A5, SND1, SNX5, SORBS2, SPTBN1, SRA1, STXBP3, TAF4B, TIAF1, TRADD, TSC2, TXLNA, U2AF1, UBE2B, UBR5, URI1, USP12, VPS37B.

Features "significantly different between the classes at 0.001 significance level" were used for class prediction of "healthy conditions vs. malignant tumor" samples including malignant tumor cases versus their statistically age-balanced Contr samples (contrast 2) using 24 samples (12 control and 12 malignant tumor samples). Using features significantly different between the classes at 0.001 significance level , for those 80 features the Compound Covariate Predictor (CCP), Diagonal Linear Discriminant Analysis (DLDA), 1-Nearest Neighbor (1NN), 3-Nearest Neighbors (3NN), Nearest Centroid (NC), and Support Vector Machines (SVM) enabled correct classification of 96% of the tested samples. Prior to feature subsetting features with less than 20% of expression data having least a 1.5 -fold change in either direction from gene's median value, a percentile of the log-ratio variation in less than 75, and the 50th Percentile of intensities with less than a value of 500 got filtered out. Feature subsetting and prediction was performed repeatedly for each of the K-fold (K=24) cross-validations of this subsetting method.

By that means the rate of correct classification was calculated.

### Performance of classifiers during cross-validation.

| | **Compound-CovariatePredic-tor** | **Diagonal Lin-earDiscriminan-tAnalysis** | **1-Nearest-Neighbor** | **3-Nearest-Neighbors** | **Near-estCen-troid** | **SupportVec-torMachines** | **BayesianCompound-CovariatePredictor** |
|---|---|---|---|---|---|---|---|
| Mean percentof correctclas-sification | 96 | 96 | 96 | 96 | 96 | 96 | 96 |

### Performance of the Compound Covariate Predictor Classifier:

| **Class** | **Sensitivity** | **Specificity** | **PPV** | **NPV** |
|---|---|---|---|---|
| **Contr** | 1 | 0.917 | 0.923 | 1 |
| **Mal** | 0917 | 1 | 1 | 0.933 |

### Performance of the Diagonal Linear Discriminant Analysis Classifier:

| **Class** | **Sensitivity** | **Specificity** | **PPV** | **NPV** |
|---|---|---|---|---|
| **Contr** | 1 | 0917 | 0.933 | 1 |
| **Mal** | 0917 | 1 | 1 | 0.933 |

### Performance of the 1-Nearest Neighbor Classifier:

| **Class** | **Sensitivity** | **Specificity** | **PPV** | **NPV** |
|---|---|---|---|---|
| **Contr** | 1 | 0917 | 0.933 | 1 |
| **Mal** | 0917 | 1 | 1 | 0.933 |

### Performance of the 3-Nearest Neighbors Classifier:

| **Class** | **Sensitivity** | **Specificity** | **PPV** | **NPV** |
|---|---|---|---|---|
| **Contr** | 1 | 0917 | 0.933 | 1 |
| **Mal** | 0917 | 1 | 1 | 0.933 |

### Performance of the Nearest Centroid Classifier:

| **Class** | **Sensitivity** | **Specificity** | **PPV** | **NPV** |
|---|---|---|---|---|
| **Contr** | 1 | 0917 | 0.933 | 1 |
| **Mal** | 0917 | 1 | 1 | 0.933 |

### Performance of the Support Vector Machine Classifier:

| **Class** | **Sensitivity** | **Specificity** | **PPV** | **NPV** |
|---|---|---|---|---|
| **Contr** | 1 | 0917 | 0.933 | 1 |
| **Mal** | 0.917 | 1 | 1 | 0.923 |

### Example 7.7: "healthy conditions vs. malign tumor" (using just run 3) - 25 greedy pairs > 100% CCP, DLDA, 1NN, 3NN, NC, SVM The following markers were identified according to this example:

**List 9:** ADAMTS16, AKR1C4, AKT1, AP2A1, ARCN1, ARHGAP1, ATP5SL, CCND1, CD81, CD97, CDC25B, CDT1, CINP, CRAT, DDX24, DOK2, EHD4, FAM208A, FGB, FLNA, FTL, FUT8, GBAS, HARS, HIVEP1, HNRNPA1, HOOK2, IST1 (includes EG:307833), KANSL2, KIF22, LRRC47, LRSAM1, MED23, MGA, MYH14, NAGLU, NUMA1, PACS1, PLCE1, PNPT1, RAB11FIP3, RAD21, RPS18, SH3BGRL3, STAB1, TAOK1, TPM3, TXLNG2P, VIM, VPS72 (includes EG:100001285).

The "25 greedy pairs" strategy was used for class prediction, and it was possible to very efficiently build classifiers for distinguishing malignant tumors versus their statistically age-balanced Contr samples (contrast 2) using 24 samples (12 control and 12 malignant tumor samples). Using "25 greedy pairs" of features on arrays, for those 50 features the Compound Covariate Predictor (CCP), Diagonal Linear Discriminant Analysis (DLDA), 1-Nearest Neighbor (1NN), 3-Nearest Neighbors (3NN), Nearest Centroid (NC), and Support Vector Machines (SVM) enabled correct classification of 100% of the tested samples.

Prior to feature subsetting features with less than 20 % of expression data having least a 1.5 -fold change in either direction from gene's median value, a percentile of the log-ratio variation in less than 75, and the 50th Percentile of intensities with less than a value of 500 got filtered out. Feature subsetting and prediction was performed repeatedly for each of the K-fold (K=24) cross-validations of this subsetting method. By that means the rate of correct classification was calculated.

### Performance of classifiers during cross-validation.

| | **Compound-CovariatePredictor** | **Diagonal LinearDiscriminantAnalysis** | **1-Nearest-Neighbor** | **3-Nearest-Neighbors** | **Near-estCentroid** | **SupportVectorMachines** | **BayesianCompound-CovariatePredictor** |
|---|---|---|---|---|---|---|---|
| Mean percentof correctclas-sification: | 100 | 100 | 100 | 100 | 100 | 100 | 100 |

### Performance of the Compound Covariate Predictor Classifier:

| **Class** | **Sensitivity** | **Specificity** | **PPV** | **NPV** |
|---|---|---|---|---|
| **Ctrl** | 1 | 1 | 1 | 1 |
| **Mal** | 1 | 1 | 1 | 1 |

### Performance of the Diagonal Linear Discriminant Analysis Classifier:

| **Class** | **Sensitivity** | **Specificity** | **PPV** | **NPV** |
|---|---|---|---|---|
| **Ctrl** | 1 | 1 | 1 | 1 |
| **Mal** | 1 | 1 | 1 | 1 |

### Performance of the 1-Nearest Neighbor Classifier:

| **Class** | **Sensitivity** | **Specificity** | **PPV** | **NPV** |
|---|---|---|---|---|
| **Ctrl** | 1 | 1 | 1 | 1 |
| **Mal** | 1 | 1 | 1 | 1 |

### Performance of the 3-Nearest Neighbors Classifier:

| **Class** | **Sensitivity** | **Specificity** | **PPV** | **NPV** |
|---|---|---|---|---|
| **Ctrl** | 1 | 1 | 1 | 1 |
| **Mal** | 1 | 1 | 1 | 1 |

### Performance of the Nearest Centroid Classifier:

| **Class** | **Sensitivity** | **Specificity** | **PPV** | **NPV** |
|---|---|---|---|---|
| **Ctrl** | 1 | 1 | 1 | 1 |
| **Mal** | 1 | 1 | 1 | 1 |

### Performance of the Support Vector Machine Classifier:

| **Class** | **Sensitivity** | **Specificity** | **PPV** | **NPV** |
|---|---|---|---|---|
| **Ctrl** | 1 | 1 | 1 | 1 |
| **Mal** | 1 | 1 | 1 | 1 |

### Example 7.8: "healthy conditions vs. malign tumor" (using just run 4) - 25 greedy pairs > 100% CCP, DLDA, 1NN, 3NN, NC, SVM

The following markers were identified according to this example:

**List 10:** ACIN1, ACTR3, ADH5 (includes EG:100145871), AMN1 (includes EG:196394), AMT, ATN1, ATRX, AURKAIP1, CDT1, CISH, CLCN2, CPNE1, CYLD, DENND1C, EIF3J, EIF3L, EZR, FBXO6, GOLM1, HLA-A, HLA-B, HSPG2, KPNB1, LAMC1, MAGED2, MAPK8IP3, NAP1L1, NDC80, NUP85 (includes EG:287830), OLFM2, OTUB1, PDCD6IP, POLR2H, PSMC4, PTK2B, PTPN7, RAC2, RAD9A, RAN, RPS17/RPS17L, RRBP1, SCG5, SCO1, SNX6, SRPR, STIP1, STX18, SUPT5H, TMOD3, ZC3H7A. The "25 greedy pairs" strategy was used for class prediction, and it was possible to very efficiently build classifiers for distinguishing malignant tumors versus their statistically age-balanced Contr samples (contrast 2) using 23 samples (11 control and 12 malignant tumor samples). Using "25 greedy pairs" of features on arrays, for those 50 features the Compound Covariate Predictor (CCP), Diagonal Linear Discriminant Analysis (DLDA), 1-Nearest Neighbor (1NN), 3-Nearest Neighbors (3NN), Nearest Centroid (NC), and Support Vector Machines (SVM) enabled correct classification of 100% of the tested samples.

Prior to feature subsetting features with less than 20 % of expression data having least a 1.5 -fold change in either direction from gene's median value, a percentile of the log-ratio variation in less than 75, and the 50th Percentile of intensities with less than a value of 500 got filtered out. Feature subsetting and prediction was performed repeatedly for each of the K-fold (K=23) cross-validations of this subsetting method. By that means the rate of correct classification was calculated.

### Performance of classifiers during cross-validation.

| | **Compound-CovariatePredic-**tor | **Diagonal LinearDiscriminantAnalysis** | **1-Nearest-Neighbor** | **3-Nearest-Neighbors** | **Near-estCen-troid** | **SupportVectorMachines** | **BayesianCompound-CovariatePredictor** |
|---|---|---|---|---|---|---|---|
| Mean percentof correctclassification: | 100 | 100 | 100 | 100 | 100 | 100 | 100 |

### Performance of the Compound Covariate Predictor Classifier:

| **Class** | **Sensitivity** | **Specificity** | **PPV** | **NPV** |
|---|---|---|---|---|
| **Ctrl** | 1 | 1 | 1 | 1 |
| **Mal** | 1 | 1 | 1 | 1 |

### Performance of the Diagonal Linear Discriminant Analysis Classifier:

| **Class** | **Sensitivity** | **Specificity** | **PPV** | **NPV** |
|---|---|---|---|---|
| **Ctrl** | 1 | 1 | 1 | 1 |
| **Mal** | 1 | 1 | 1 | 1 |

### Performance of the 1-Nearest Neighbor Classifier:

| **Class** | **Sensitivity** | **Specificity** | **PPV** | **NPV** |
|---|---|---|---|---|
| **Ctrl** | 1 | 1 | 1 | 1 |
| **Mal** | 1 | 1 | 1 | 1 |

### Performance of the 3-Nearest Neighbors Classifier:

| **Class** | **Sensitivity** | **Specificity** | **PPV** | **NPV** |
|---|---|---|---|---|
| **Ctrl** | 1 | 1 | 1 | 1 |
| **Mal** | 1 | 1 | 1 | 1 |

### Performance of the Nearest Centroid Classifier:

| **Class** | **Sensitivity** | **Specificity** | **PPV** | **NPV** |
|---|---|---|---|---|
| **Ctrl** | 1 | 1 | 1 | 1 |
| **Mal** | 1 | 1 | 1 | 1 |

### Performance of the Support Vector Machine Classifier:

| **Class** | **Sensitivity** | **Specificity** | **PPV** | **NPV** |
|---|---|---|---|---|
| **Ctrl** | 1 | 1 | 1 | 1 |
| **Mal** | 1 | 1 | 1 | 1 |

### Example 7.9: "healthy conditions vs. malignant tumor" (using just run 5) - p<0.001 > 100% DLDA

The following markers were identified according to this example: **List 11:** AARS, ACTN4, AMBRA1, ANTXR1, ANXA6, APBB1, ARHGAP44, ATXN1, BAZ1B, C12orf35, CAMSAP1, CHMP1B, CTSH, DDX39A, DFFA, EEF1D, FBRS, FBXL5, FLNA, GART, GGA3, GLUL, GMPPB, GNAI2, HLA-C, HSPA1A/HSPA1B, HSPB1, HUWE1, IGF2R, LUC7L3, MAP3K3, MCM3AP, MGEA5, MINK1, MTA2, MUC2 (includes EG:4583), NISCH, NLE1, PACS1, PCDH7, PDXK, PJA2, PLXNA1, PSMD13, PTPRF, RABEPK, RNF213, RPLP0P2, RPS6KB2, SBF1, SEC13, SEPT1, SEPT6, SLC25A6, SNX4, SPNS2, STAT1, TAX1BP1, THAP7, TMSB10/TMSB4X, TNFSF13, TPR, TRIP12, TYK2, UBA52, UBR4, UQCRC1.

Features "significantly different between the classes at 0.001 significance level" were used for class prediction of "healthy conditions vs. malignant tumor" samples including malignant tumor cases versus their statistically age-balanced Contr samples (contrast 2) using 22 samples (10 control and 12 malignant tumor samples). Using features significantly different between the classes at 0.001 significance level, for those 67 features the Diagonal Linear Discriminant Analysis (DLDA) enabled correct classification of 100% of the tested samples. Prior to feature subsetting features with less than 20 % of expression data having least a 1.5 -fold change in either direction from gene's median value, a percentile of the log-ratio variation in less than 75, and the 50th Percentile of intensities with less than a value of 500 got filtered out. Feature subsetting and prediction was performed repeatedly for each of the K-fold (K=22) cross-validations of this subsetting method. By that means the rate of correct classification was calculated.

### Performance of classifiers during cross-validation.

| | **Compound-CovariatePredictor** | **Diagonal LinearDiscriminantAnalysis** | **1-Nearest-Neighbor** | **3-Nearest-Neighbors** | **NearestCentroid** | **SupportVectorMachines** | **BayesianCompound-CovariatePredictor** |
|---|---|---|---|---|---|---|---|
| Mean percentof correctclas-sification: | 95 | 100 | 91 | 91 | 91 | 95 | 95 |

### Performance of the Diagonal Linear Discriminant Analysis Classifier:

| **Class** | **Sensitivity** | **Specificity** | **PPV** | **NPV** |
|---|---|---|---|---|
| **Ctrl** | 1 | 1 | 1 | 1 |
| **Mal** | 1 | 1 | 1 | 1 |

### Example 7.10: "healthy conditions vs. malign tumor" (using just run 6) - 25 greedy pairs > 96% CCP, DLDA, 1NN, 3NN, NC, SVM The following markers were identified according to this example:

**List 12:** ACAT2, ADAMTS10, AFMID, AGAP6 (includes others), APBB1IP, ARHGEF11, ATIC, BCL11A, BEX4, C8orf37, CACNB3, CLU, CO-RO2B, CPNE6, CTBP2, EGR1, EIF3G, HSPG2, KLHDC3, LCK, MAP3K4, MCM7, MMP14, NEFM, NOC2L, NONO, PCDH9, PLEKHH3, PMS2, PTPRA, RARS, RGS2 (includes EG:19735), RNF146, RPL10, SF3A3, SH3BP1, SNX6, STMN4, STRN4, TPM2, TSEN2, TTYH1, VASP, VAV2, WARS, WDR6, WDTC1, ZC3H13, ZFP2, ZNF7.The "25 greedy pairs" strategy was used for class prediction, and it was possible to very efficiently build classifiers for distinguishing malignant tumors versus their statistically age-balanced Contr samples (contrast 2) using 23 samples (5 control and 18 malignant tumor samples). Using "25 greedy pairs" of features on arrays, for those 50 features the Compound Covariate Predictor (CCP), Diagonal Linear Discriminant Analysis (DLDA), 1-Nearest Neighbor (1NN), 3-Nearest Neighbors (3NN), Nearest Centroid (NC), and Support Vector Machines (SVM) enabled correct classification of 96% of the tested samples.

Prior to feature subsetting features with less than 20 % of expression data having least a 1.5 -fold change in either direction from gene's median value, a percentile of the log-ratio variation in less than 75, and the 50th Percentile of intensities with less than a value of 500 got filtered out. Feature subsetting and prediction was performed repeatedly for each of the K-fold (K=23) cross-validations of this subsetting method. By that means the rate of correct classification was calculated.

### Performance of classifiers during cross-validation.

| | **Compound-CovariatePredictor** | **Diagonal LinearDiscriminantAnalysis** | **1-NearestNeigh bor** | **3-NearestNeigh bors** | **NearestCentroid** | **SupportVec portVectorMachines** | **BayesianCompoundCovariatePredictor** |
|---|---|---|---|---|---|---|---|
| Mean percentof correctclassification: | 96 | 96 | 96 | 96 | 96 | 96 | 96 |

### Performance of the Compound Covariate Predictor Classifier:

| **Class** | **Sensitivity** | **Specificity** | **PPV** | **NPV** |
|---|---|---|---|---|
| **Ctrl** | 0.8 | 1 | 1 | 0.947 |
| **Mal** | 1 | 0.8 | 0.947 | 1 |

### Performance of the Diagonal Linear Discriminant Analysis Classifier:

| **Class** | **Sensitivity** | **Specificity** | **PPV** | **NPV** |
|---|---|---|---|---|
| **Ctrl** | 0.8 | 1 | 1 | 0.947 |
| **Mal** | 1 | 0.8 | 0.947 | 1 |

### Performance of the 1-Nearest Neighbor Classifier:

| **Class** | **Sensitivity** | **Specificity** | **PPV** | **NPV** |
|---|---|---|---|---|
| **Ctrl** | 0.8 | 1 | 1 | 0.947 |
| **Mal** | 1 | 0.8 | 0.947 | 1 |

### Performance of the 3-Nearest Neighbors Classifier:

| **Class** | **Sensitivity** | **Specificity** | **PPV** | **NPV** |
|---|---|---|---|---|
| **Ctrl** | 0.8 | 1 | 1 | 0.947 |
| **Mal** | 1 | 0.8 | 0.947 | 1 |

### Performance of the Nearest Centroid Classifier:

| **Class** | **Sensitivity** | **Specificity** | **PPV** | **NPV** |
|---|---|---|---|---|
| **Ctrl** | 0.8 | 1 | 1 | 0.947 |
| **Mal** | 1 | 0.8 | 0.947 | 1 |

### Performance of the Support Vector Machine Classifier:

| **Class** | **Sensitivity** | **Specificity** | **PPV** | **NPV** |
|---|---|---|---|---|
| **Ctrl** | 0.8 | 1 | 1 | 0.947 |
| **Mal** | 1 | 0.8 | 0.947 | 1 |

### Example 7.11: "benign tumor vs. malign tumor" (using just run 1) - 25 greedy pairs > 79% DLDA, 1NN, SVM

The following markers were identified according to this example: **List 13:** ABCA11P, AKAP9, ARID4B, ATG16L1, ATXN2, BCAM, C17orf56, CCDC149, CCL28, CEP57, CIC, CNTROB, COL3A1, DMPK, EGR1, FLOT1, FOXP3, GLYR1, GNAS, GOLGA7, HMOX2, IGLC1, IRF1 (includes EG:16362), KIAA1244, LPXN, MORF4L1, NCOA4, NRG3, NXPH3, PARD3, PDZD4, PRKCD, RASGRP1, RCOR2, RRM1, SAMD14, SAP18, SFN, SSBP2, TAF7, THOP1, TNFRSF6B, TRIM44, TXNDC5, UTY, VPS72 (includes EG:100001285), WDR33, WDR47, WDR73, ZNF638.

The "25 greedy pairs" strategy was used for class prediction, and it was possible to very efficiently build classifiers for distinguishing malignant tumors versus their statistically age-balanced benign tumor samples (contrast 3) using 24 samples (12 benign and 12 malignant tumor samples). Using "25 greedy pairs" of features on arrays, for those 50 features the Diagonal Linear Discriminant Analysis (DLDA), 1-Nearest Neighbor (1NN), and Support Vector Machines (SVM) enabled correct classification of 79% of the tested samples.

Prior to feature subsetting features with less than 20 % of expression data having least a 1.5 -fold change in either direction from gene's median value, a percentile of the log-ratio variation in less than 75, and the 50th Percentile of intensities with less than a value of 500 got filtered out. Feature subsetting and prediction was performed repeatedly for each of the K-fold (K=24) cross-validations of this subsetting method. By that means the rate of correct classification was calculated.

### Performance of classifiers during cross-validation.

| | **Compound-CovariatePredictor** | **Diagonal LinearDiscriminantAnalysis** | **1-Nearest-Neighbor** | **3-Nearest-Neighbors** | **NearestCentroid** | **SupportVectorMachines** | **BayesianCompound-CovariatePredictor** |
|---|---|---|---|---|---|---|---|
| Mean percentof correctclassification: | 75 | 79 | 79 | 75 | 75 | 79 | 78 |

### Performance of the Diagonal Linear Discriminant Analysis Classifier:

| **Class** | **Sensitivity** | **Specificity** | **PPV** | **NPV** |
|---|---|---|---|---|
| **Ben** | 0.667 | 0.917 | 0.889 | 0.733 |
| **Mal** | 0.917 | 0.667 | 0.733 | 0.889 |

### Performance of the 1-Nearest Neighbor Classifier:

| **Class** | **Sensitivity** | **Specificity** | **PPV** | **NPV** |
|---|---|---|---|---|
| **Ben** | 0.667 | 0.917 | 0.889 | 0.733 |
| **Mal** | 0.917 | 0.667 | 0.733 | 0.889 |

### Performance of the Support Vector Machine Classifier:

| **Class** | **Sensitivity** | **Specificity** | **PPV** | **NPV** |
|---|---|---|---|---|
| **Ben** | 0.75 | 0.833 | 0.818 | 0.769 |
| **Mal** | 0.833 | 0.75 | 0.769 | 0.818 |

### Example 7.12: "benign tumor vs. malign tumor" (using just run 2) - 25 greedy pairs > 78% CCP, DLDA, 1NN, 3NN, NC, SVM The following markers were identified according to this example:

**List 14:** A2M, ABI1, AIDA, ANXA11, ARAP1, ARHGEF40, ARL8A, BZW2, C12orf45, C21orf2, CHD1L, CSRNP1, DBNL, EIF4G1, ERP29, EVPL, FAM192A, GABARAPL2, GOLGA2, HK1, HMGB2, HNRNPA3, HTATIP2, KHDRBS1, LARP1, LSP1 (includes EG:16985), MICAL3, NEDD4L, PCGF2, PKP3, PLCB3, POLR3E, POTEE/POTEF, PSMB3, R3HDM4, RAB11FIP3, RGS2 (includes EG:19735), RIPK1, SAFB2, SLC7A5, SMAP2, SMCR8, SNX5, SPHK2, SURF4, THAP7, TNRC18, TRAFD1, WASH1/WASH5P, ZNF839.

The "25 greedy pairs" strategy was used for class prediction, and it was possible to very efficiently build classifiers for distinguishing malignant tumors versus their statistically age-balanced benign tumor samples (contrast 3) using 23 samples (11 benign and 12 malignant tumor samples). Using "25 greedy pairs" of features on arrays, for those 50 features the Compound Covariate Predictor (CCP), Diagonal Linear Discriminant Analysis (DLDA), 1-Nearest Neighbor (1NN), 3-Nearest Neighbors (3NN), Nearest Centroid (NC), and Support Vector Machines (SVM) enabled correct classification of 78% of the tested samples.

Prior to feature subsetting features with less than 20 % of expression data having least a 1.5 -fold change in either direction from gene's median value, a percentile of the log-ratio variation in less than 75, and the 50th Percentile of intensities with less than a value of 500 got filtered out. Feature subsetting and prediction was performed repeatedly for each of the K-fold (K=23) cross-validations of this subsetting method. By that means the rate of correct classification was calculated.

### Performance of classifiers during cross-validation.

| | **Compound-CovariatePredictor** | **Diagonal LinearDiscriminantAnalysis** | **1-NearestNeigh bor** | **3-NearestNeigh bors** | **NearestCentroid** | **SupportVec portVectorMachines** | **BayesianCompoundCovariatePredictor** |
|---|---|---|---|---|---|---|---|
| Mean percentof correctclassification: | 78 | 78 | 78 | 78 | 78 | 78 | 78 |

### Performance of the Compound Covariate Predictor Classifier:

| **Class** | **Sensitivity** | **Specificity** | **PPV** | **NPV** |
|---|---|---|---|---|
| **Ben** | 0.727 | 0.833 | 0.8 | 0.769 |
| **Mal** | 0.833 | 0.727 | 0.769 | 0.8 |

### Performance of the Diagonal Linear Discriminant Analysis Classifier:

| **Class** | **Sensitivity** | **Specificity** | **PPV** | **NPV** |
|---|---|---|---|---|
| **Ben** | 0.727 | 0.833 | 0.8 | 0.769 |
| **Mal** | 0.833 | 0.727 | 0.769 | 0.8 |

### Performance of the 1-Nearest Neighbor Classifier:

| **Class** | **Sensitivity** | **Specificity** | **PPV** | **NPV** |
|---|---|---|---|---|
| **Ben** | 0.727 | 0.833 | 0.8 | 0.769 |
| **Mal** | 0.833 | 0.727 | 0.769 | 0.8 |

### Performance of the 3-Nearest Neighbors Classifier:

| **Class** | **Sensitivity** | **Specificity** | **PPV** | **NPV** |
|---|---|---|---|---|
| **Ben** | 0.727 | 0.833 | 0.8 | 0.769 |
| **Mal** | 0.833 | 0.727 | 0.769 | 0.8 |

### Performance of the Nearest Centroid Classifier:

| **Class** | **Sensitivity** | **Specificity** | **PPV** | **NPV** |
|---|---|---|---|---|
| **Ben** | 0.727 | 0.833 | 0.8 | 0.769 |
| **Mal** | 0.833 | 0.727 | 0.769 | 0.8 |

### Performance of the Support Vector Machine Classifier:

| **Class** | **Sensitivity** | **Specificity** | **PPV** | **NPV** |
|---|---|---|---|---|
| **Ben** | 0.727 | 0.833 | 0.8 | 0.769 |
| **Mal** | 0.833 | 0.727 | 0.769 | 0.8 |

### Example 7.13: "benign tumor vs. malign tumor" (using just run 3) - 25 greedy pairs > 96% CCP, 3NN, NC

The following markers were identified according to this example: **List 15:** ACTR1B, AKR1C4, APEX1, ARCN1, ATP5D, ATP5SL, BZRAP1, C17orf70, C19orf25, CCND1, CDC25B, CDC37, CNTD1, CTSD, DHX16, DHX9, EZR, FIGNL1, FLNA, FUT8, FZR1, HNRNPUL1, KCNJ14, KHDRBS1, KLF5, LOC440354, LRRC47, MAPRE1, MEGF8, MYO9A, NAP1L1, NCOA4, P4HB, PACS1, PEF1, PIP5K1A, POLI, PPM1G, RCBTB1, RSL24D1, SIRT2, SPOCK2, STAT6, STK10, SURF6, TALDO1, TXLNG2P, UNC45A, VPS72 (includes EG:100001285), ZNF202.

The "25 greedy pairs" strategy was used for class prediction, and it was possible to very efficiently build classifiers for distinguishing malignant tumors versus their statistically age-balanced benign tumor samples (contrast 3) using 24 samples (12 benign and 12 malignant tumor samples). Using "25 greedy pairs" of features on arrays, for those 50 features the Compound Covariate Predictor (CCP), 3-Nearest Neighbors (3NN), and Nearest Centroid (NC) enabled correct classification of 96% of the tested samples.

Prior to feature subsetting features with less than 20 % of expression data having least a 1.5 -fold change in either direction from gene's median value, a percentile of the log-ratio variation in less than 75, and the 50th Percentile of intensities with less than a value of 500 got filtered out. Feature subsetting and prediction was performed repeatedly for each of the K-fold (K=24) cross-validations of this subsetting method. By that means the rate of correct classification was calculated.

### Performance of classifiers during cross-validation.

| | **Compound-CovariatePredictor** | **Diagonal LinearDiscriminantAnalysis** | **1-Nearest-Neighbor** | **3-Nearest-Neighbors** | **NearestCentroid** | **SupportVectorMachines** | **BayesianCompound-CovariatePredictor** |
|---|---|---|---|---|---|---|---|
| Mean percentof correctclassification: | 96 | 92 | 92 | 96 | 96 | 92 | 96 |

### Performance of the Compound Covariate Predictor Classifier:

| **Class** | **Sensitivity** | **Specificity** | **PPV** | **NPV** |
|---|---|---|---|---|
| **Ben** | 1 | 0.917 | 0.923 | 1 |
| **Mal** | 0.917 | 1 | 1 | 0.923 |

### Performance of the 3-Nearest Neighbors Classifier:

| **Class** | **Sensitivity** | **Specificity** | **PPV** | **NPV** |
|---|---|---|---|---|
| **Ben** | 1 | 0.917 | 0.923 | 1 |
| **Mal** | 0.917 | 1 | 1 | 0.923 |

### Performance of the Nearest Centroid Classifier:

| **Class** | **Sensitivity** | **Specificity** | **PPV** | **NPV** |
|---|---|---|---|---|
| **Ben** | 1 | 0.917 | 0.923 | 1 |
| **Mal** | 0.917 | 1 | 1 | 0.923 |

### Example 7.14: "benign tumor vs. malignant tumor" (using just run 4) - p<0.001 > 100% 1NN, SVM The following markers were identified according to this example:

**List 16:**AMICA1, ARF5, CECR5, CPNE1, CSF1R, CUEDC2, CUL1, GON4L, ITFG3, LCMT2, MAGED2, MCM5, METTL3, POLR2H, POLR2L, PSME4, RNF34, RRBP1, SND1, STAT4, TSC2, WAC, XRCC1 (includes EG:22594. Features "significantly different between the classes at 0.001 significance level" were used for class prediction of "benign tumor vs. malignant tumor" samples including malignant tumors versus their statistically age-balanced benign tumor samples (contrast 3) using 19 samples (7 benign and 12 malignant tumor samples). Using features significantly different between the classes at 0.001 significance level, for those 23 features the 1-Nearest Neighbor (1NN) and Support Vector Machines (SVM) enabled correct classification of 100% of the tested samples. Prior to feature subsetting features with less than 20 % of expression data having least a 1.5 -fold change in either direction from gene's median value, a percentile of the log-ratio variation in less than 75, and the 50th Percentile of intensities with less than a value of 500 got filtered out. Feature subsetting and prediction was performed repeatedly for each of the K-fold (K=19) cross-validations of this subsetting method. By that means the rate of correct classification was calculated.

### Performance of classifiers during cross-validation.

| | **Compound-CovariatePredictor** | **Diagonal LinearDiscriminantAnalysis** | **1-Nearest-Neighbor** | **3-Nearest-Neighbors** | **NearestCentroid** | **SupportVectorMachines** | **BayesianCompound-CovariatePredictor** |
|---|---|---|---|---|---|---|---|
| Mean percentof correctclassification: | 79 | 79 | 100 | 84 | 84 | 100 | 83 |

### Performance of the 1-Nearest Neighbor Classifier:

| **Class** | **Sensitivity** | **Specificity** | **PPV** | **NPV** |
|---|---|---|---|---|
| **Ben** | 1 | 1 | 1 | 1 |
| **Mal** | 1 | 1 | 1 | 1 |

### Performance of the Support Vector Machine Classifier:

| **Class** | **Sensitivity** | **Specificity** | **PPV** | **NPV** |
|---|---|---|---|---|
| **Ben** | 1 | 1 | 1 | 1 |
| **Mal** | 1 | 1 | 1 | 1 |

### Example 7.15: "healthy conditions vs. hereditary breast cancer" (using just run 5) - p<0.001 > 95% CCP, DLDA, 1NN, 3NN, NC, SVM

The following markers were identified according to this example:

List 17: BAZ1B, BIN3, EHMT1, KIFC3, LRBA, NAPA, NEU1, PACS1, PJA2, PLBD1, PLOD1, PLXNA3, PPIF, PRDX6, TSC22D3, WDR75. Features "significantly different between the classes at 0.001 significance level" were used for class prediction of "healthy conditions vs. hereditary breast cancer" samples including hereditary tumors versus their statistically age-balanced control samples (contrast 4) using 22 samples (10 control and 12 hereditary tumor samples). Using features significantly different between the classes at 0.001 significance level, for those 22 features the Compound Covariate Predictor (CCP), Diagonal Linear Discriminant Analysis (DLDA), 1-Nearest Neighbor (1NN), 3-Nearest Neighbors (3NN), Nearest Centroid (NC), and Support Vector Machines (SVM) enabled correct classification of 95% of the tested samples. Prior to feature subsetting features with less than 20 % of expression data having least a 1.5 -fold change in either direction from gene's median value, a percentile of the log-ratio variation in less than 75, and the 50th Percentile of intensities with less than a value of 500 got filtered out. Feature subsetting and prediction was performed repeatedly for each of the K-fold (K=22) cross-validations of this subsetting method. By that means the rate of correct classification was calculated.

### Performance of classifiers during cross-validation.

| | **Compound-CovariatePredic-tor** | **Diagonal LinearDiscrimi-nantAnalysis** | **1-Nearest-Neighbor** | **3-Nearest-Neighbors** | **NearestCen-troid** | **SupportVectorMachines** | **BayesianCompound-CovariatePredictor** |
|---|---|---|---|---|---|---|---|
| Mean percentof correctclassification: | 95 | 95 | 95 | 95 | 95 | 95 | 95 |

### Performance of the Compound Covariate Predictor Classifier:

| **Class** | **Sensitivity** | **Specificity** | **PPV** | **NPV** |
|---|---|---|---|---|
| **Ctrl** | 09 | 1 | 1 | 0.923 |
| **Her** | 1 | 0.9 | 0.923 | 1 |

### Performance of the Diagonal Linear Discriminant Analysis Classifier:

| **Class** | **Sensitivity** | **Specificity** | **PPV** | **NPV** |
|---|---|---|---|---|
| **Ctrl** | 0.9 | 1 | 1 | 0.923 |
| **Her** | 1 | 0.9 | 0.923 | 1 |

### Performance of the 1-Nearest Neighbor Classifier:

| **Class** | **Sensitivity** | **Specificity** | **PPV** | **NPV** |
|---|---|---|---|---|
| **Ctrl** | 0.9 | 1 | 1 | 0.923 |
| **Her** | 1 | 0.9 | 0.923 | 1 |

### Performance of the 3-Nearest Neighbors Classifier:

| **Class** | **Sensitivity** | **Specificity** | **PPV** | **NPV** |
|---|---|---|---|---|
| **Ctrl** | 0.9 | 1 | 1 | 0.923 |
| **Her** | 1 | 0.9 | 0.923 | 1 |

### Performance of the Nearest Centroid Classifier:

| **Class** | **Sensitivity** | **Specificity** | **PPV** | **NPV** |
|---|---|---|---|---|
| **Ctrl** | 0.9 | 1 | 1 | 0.923 |
| **Her** | 1 | 0.9 | 0.923 | 1 |

### Performance of the Support Vector Machine Classifier:

| **Class** | **Sensitivity** | **Specificity** | **PPV** | **NPV** |
|---|---|---|---|---|
| **Ctrl** | 0.9 | 1 | 1 | 0.923 |
| **Her** | 1 | 0.9 | 0.923 | 1 |

### Example 7.16: "healthy conditions vs. hereditary breast cancer" (using just run 6) - 25 greedy pairs > 71% CCP, DLDA, NC

The following markers were identified according to this example: **List 18**:ANXA6, ATIC, BIN1, BIN3, CBX4, CCDC51, CCNL2, CDC37, CUL7, DCAF6, DSP, ENTPD6, ETFA, EXOSC10, EZH1, HEXDC, HMGN2, HSP90B1, IGHG1, IK, ILF3, IMMT, IMP4, KAT2A, KCMF1, LDHB (includes EG:3945), LOC284023, LTBP3, MYCL1, NEFL, NUDT16L1, PLCB3, PLEKHA4, PLEKHO1, PPID, PPP1R9B, PSMB6, PTPRE, RCL1, RECQL4, RNF146, RPL37A, RPL7, SCAF1, SMARCC2, STX11, SWI5, TRO, VIMP. The "25 greedy pairs" strategy was used for class prediction, and it was possible to very efficiently build classifiers for distinguishing hereditary tumors versus their statistically age-balanced benign tumor samples (contrast 4) using 17 samples (5 control and 12 hereditary tumor samples). Using "25 greedy pairs" of features on arrays, for those 50 features the Compound Covariate Predictor (CCP), Diagonal Discriminant Analysis (DLDA), and Nearest Centroid (NC) enabled correct classification of 71% of the tested samples.

Prior to feature subsetting features with less than 20 % of expression data having least a 1.5 -fold change in either direction from gene's median value, a percentile of the log-ratio variation in less than 75, and the 50th Percentile of intensities with less than a value of 500 got filtered out. Feature subsetting and prediction was performed repeatedly for each of the K-fold (K=17) cross-validations of this subsetting method. By that means the rate of correct classification was calculated.

### Performance of classifiers during cross-validation.

| | **CompoundCovariatePredictor** | **Diagonal Line- arDiscriminantAnalysis** | **1-Nearest-Neighbor** | **3-Nearest-Neighbors** | **Nearest-Centroid** | **SupportVectorMachines** | **BayesianCompound-CovariatePredictor** |
|---|---|---|---|---|---|---|---|
| Mean percentof correctclassification: | 71 | 71 | 59 | 59 | 71 | 59 | 67 |

### Performance of the Compound Covariate Predictor Classifier:

| **Class** | **Sensitivity** | **Specificity** | **PPV** | **NPV** |
|---|---|---|---|---|
| **Ctrl** | 0.4 | 0.833 | 0.5 | 0.769 |
| **Her** | 0.833 | 0.4 | 0.769 | 0.5 |

### Performance of the Diagonal Linear Discriminant Analysis Classifier:

| **Class** | **Sensitivity** | **Specificity** | PPV | NPV |
|---|---|---|---|---|
| **Ctrl** | 0.4 | 0.833 | 0.5 | 0.769 |
| **Her** | 0.833 | 0.4 | 0.769 | 0.5 |

### Performance of the Nearest Centroid Classifier:

| **Class** | **Sensitivity** | **Specificity** | **PPV** | **NPV** |
|---|---|---|---|---|
| **Ctrl** | 0.4 | 0.833 | 0.5 | 0.769 |
| **Her** | 0.833 | 0.4 | 0.769 | 0.5 |

### Example 7.17: "malign tumor vs. hereditary breast cancer" (using just run 5) - 25 greedy pairs > 100% CCP, DLDA, 1NN, 3NN, NC, SVM The following markers were identified according to this example:

**List 19:** ACTN4, AIP, AMBRA1, ANXA6, ARHGAP44, BIN3, BNIP1, BTBD7, C3orf19, CTSH, DFFA, EHMT1, EIF4B, FAM69B, GLUL, HLA-C, HNRNPA0, HNRNPK, HNRPDL, HSPA1A/HSPA1B, IGF2R, IK, KCNK5, KIFC3, LGALS3, LONP1, MCM3AP, METTL17, MSLN, NAPA, NISCH, PDE2A, PLOD1, PLXNA3, PRDX6, PTPRF, PUF60, PXN, RNF213, RNF219, RPLP0P2, RSPRY1, SAT2, SBF1, SEPT1, SLC7A5, SSRP1, TBC1D9B, TRIP12, ZSWIM4.

The "25 greedy pairs" strategy was used for class prediction, and it was possible to very efficiently build classifiers for distinguishing hereditary tumors versus their statistically age-balanced malignant tumor samples (contrast 5) using 24 samples 12 malignant and 12 hereditary tumor samples). Using "25 greedy pairs" of features on arrays, for those 50 features the Compound Covariate Predictor (CCP), Diagonal Discriminant Analysis (DLDA), 1-Nearest Neighbor (1NN), 3-Nearest Neighbors (3NN), Nearest Centroid (NC), and Support Vector Machines (SVM) enabled correct classification of 100% of the tested samples.

Prior to feature subsetting features with less than 20 % of expression data having least a 1.5 -fold change in either direction from gene's median value, a percentile of the log-ratio variation in less than 75, and the 50th Percentile of intensities with less than a value of 500 got filtered out. Feature subsetting and prediction was performed repeatedly for each of the K-fold (K=24) cross-validations of this subsetting method. By that means the rate of correct classification was calculated.

### Performance of classifiers during cross-validation.

| | **Compound-CovariatePredictor** | **Diagonal LinearDiscriminantAnalysis** | **1-Nearest-Neighbor** | **3-Nearest-Neighbors** | **NearestCentroid** | **SupportVectorMachines** | **BayesianCompound-CovariatePredictor** |
|---|---|---|---|---|---|---|---|
| Mean percentof correctclassification: | 100 | 100 | 100 | 100 | 100 | 100 | 100 |

### Performance of the Compound Covariate Predictor Classifier:

| **Class** | **Sensitivity** | **Specificity** | **PPV** | **NPV** |
|---|---|---|---|---|
| **Her** | 1 | 1 | 1 | 1 |
| **Mal** | 1 | 1 | 1 | 1 |

### Performance of the Diagonal Linear Discriminant Analysis Classifier:

| **Class** | **Sensitivity** | **Specificity** | **PPV** | **NPV** |
|---|---|---|---|---|
| **Her** | 1 | 1 | 1 | 1 |
| **Mal** | 1 | 1 | 1 | 1 |

### Performance of the 1-Nearest Neighbor Classifier:

| **Class** | **Sensitivity** | **Specificity** | **PPV** | **NPV** |
|---|---|---|---|---|
| **Her** | 1 | 1 | 1 | 1 |
| **Mal** | 1 | 1 | 1 | 1 |

### Performance of the 3-Nearest Neighbors Classifier:

| **Class** | **Sensitivity** | **Specificity** | **PPV** | **NPV** |
|---|---|---|---|---|
| **Her** | 1 | 1 | 1 | 1 |
| **Mal** | 1 | 1 | 1 | 1 |

### Performance of the Nearest Centroid Classifier:

| **Class** | **Sensitivity** | **Specificity** | **PPV** | **NPV** |
|---|---|---|---|---|
| **Her** | 1 | 1 | 1 | 1 |
| **Mal** | 1 | 1 | 1 | 1 |

### Performance of the Support Vector Machine Classifier:

| **Class** | **Sensitivity** | **Specificity** | **PPV** | **NPV** |
|---|---|---|---|---|
| **Her** | 1 | 1 | 1 | 1 |
| **Mal** | 1 | 1 | 1 | 1 |

### Example 7.18: "malign tumor vs. hereditary breast cancer" (using just run 6) - 25 greedy pairs > 100% CCP, DLDA, 1NN, 3NN, NC, SVM The following markers were identified according to this example:

**List 20**:ACAT2, ADAMTS10, AGAP2, AKAP17A, ANGPTL2, AP2M1, ARL4D, BCL11A, CANX, CDT1, CELSR1, CORO2B, D2HGDH, EIF3G, FLII, HIST3H2A, HLA-C, HNRNPH1, HSPA9, IK, INPPL1, LCK, MAP3K4, MTA2, NEFM, NOC2L, NRBP2, NUP93, PA2G4, PCDH9, PGD, PGRMC1, PLEKHH3, POLR2B, PTPRA, PTPRE, PUF60, RBM10, RNF34, SLU7 (includes EG:10569), SPTAN1, ST3GAL3, STOML2, TF, TSEN2, TTC3, ZNF132, ZNF300, ZNF431, ZNF7. The "25 greedy pairs" strategy was used for class prediction, and it was possible to very efficiently build classifiers for distinguishing hereditary tumors versus their statistically age- balanced malignant tumor samples (contrast 5) using 30 samples 18 malignant and 12 hereditary tumor samples). Using "25 greedy pairs" of features on arrays, for those 50 features the Compound Covariate Predictor (CCP), Diagonal Discriminant Analysis (DLDA), 1-Nearest Neighbor (1NN), 3-Nearest Neighbors (3NN), Nearest Centroid (NC), and Support Vector Machines (SVM) enabled correct classification of 100% of the tested samples.

Prior to feature subsetting features with less than 20 % of expression data having least a 1.5 -fold change in either direction from gene's median value, a percentile of the log-ratio variation in less than 75, and the 50th Percentile of intensities with less than a value of 500 got filtered out. Feature subsetting and prediction was performed repeatedly for each of the K-fold (K=30) cross-validations of this subsetting method. By that means the rate of correct classification was calculated.

### Performance of classifiers during cross-validation.

| | **CompoundCovariatePredictor** | **Diagonal LinearDiscriminantAnalysis** | **1-Nearest-Neighbor** | **3-Nearest-Neighbors** | **NearestCentroid** | **SupportVectorMachines** | **BayesianCompound-CovariatePredictor** |
|---|---|---|---|---|---|---|---|
| Mean percentof correctclassification: | 100 | 100 | 100 | 100 | 100 | 100 | 100 |

### Performance of the Compound Covariate Predictor Classifier:

| **Class** | **Sensitivity** | **Specificity** | **PPV** | **NPV** |
|---|---|---|---|---|
| **Her** | 1 | 1 | 1 | 1 |
| **Mal** | 1 | 1 | 1 | 1 |

### Performance of the Diagonal Linear Discriminant Analysis Classifier: Class Sensitivity Specificity PPV NPV

| **Class** | **Sensitivity** | **Specificity** | **PPV** | **NPV** |
|---|---|---|---|---|
| **Her** | 1 | 1 | 1 | 1 |
| **Mal** | 1 | 1 | 1 | 1 |

### Performance of the 1-Nearest Neighbor Classifier:

| **Class** | **Sensitivity** | **Specificity** | **PPV** | **NPV** |
|---|---|---|---|---|
| **Her** | 1 | 1 | 1 | 1 |
| **Mal** | 1 | 1 | 1 | 1 |

### Performance of the 3-Nearest Neighbors Classifier:

| **Class** | **Sensitivity** | **Specificity** | **PPV** | **NPV** |
|---|---|---|---|---|
| **Her** | 1 | 1 | 1 | 1 |
| **Mal** | 1 | 1 | 1 | 1 |

### Performance of the Nearest Centroid Classifier:

| **Class** | **Sensitivity** | **Specificity** | **PPV** | **NPV** |
|---|---|---|---|---|
| **Her** | 1 | 1 | 1 | 1 |
| **Mal** | 1 | 1 | 1 | 1 |

### Performance of the Support Vector Machine Classifier:

| **Class** | **Sensitivity** | **Specificity** | **PPV** | **NPV** |
|---|---|---|---|---|
| **Her** | 1 | 1 | 1 | 1 |
| **Mal** | 1 | 1 | 1 | 1 |

### Example 8: Random selection of subsets of classifiers

To exemplify the diagnostic potential of subsets from classifiers elucidated here, 10 markers were randomly chosen: Set A: CD81, HARS, CCND1, LRRC47, ATP5SL, GBAS, PNPT1, IST1 (includes EG:307833), MED23, CDT1; Set B: HARS, KIF22, VPS72 (includes EG:100001285), ARHGAP1, DDX24, HIVEP1, STAB1, IST1 (includes EG:307833), RAD21, VIM; from e.g. the 50 classifiers markers depicted after QNORM for distinguishing patients with malign tumors (n=12) versus controls (n=12) (contrast 1). Then class prediction analysis was conducted and elucidated with the random sets A) 100%, and B) 96% correct classification by the 1-Nearest Neighbor Classifier (1NN); - originally the 1NN classifier derived from the 50 genes enabled 100% correct classification. This confirms that classifiers from the present gene-lists have high potential for diagnostics, and that subsets of the classifiers panels as well as single classifiers are of high potential value for diagnostics.

For exemplification of the value of subsets of classifier markers from the panels defined in that application, the 50 classifier genes for distinguishing malign tumor vs. controls were selected and randomly chosen subsets of 2-49 markers (x-axis) were selected 1000-times. Then the classification success obtained by that subsets using the nearest centroid classifier algorithm was calculated and median % misclassification is depicted on the y-axis (Fig 2).

The 50 classifier markers for distinguishing malign tumor vs. benign tumor were selected and randomly chosen subsets of 2-49 markers (x-axis) were selected 1000-times. Then the classification success obtained by that subset using the nearest centroid classifier algorithm was calculated and median % misclassification is depicted on the y-axis (Fig 3).

## Claims

1. Method of diagnosing breast cancer in a patient by detecting the following marker proteins or a selection of at least 2 or least 20 % of the marker proteins in list 2 selected from ACAT2, ACTN4, ADAMTS10, AMBRA1, ANXA6, ARHGAP44, ATIC, CCND1, CDT1, CO-RO2B, CTSH, DFFA, E4F1, EHMT1, EIF3G, FAM208A, FLNA, GLUL, HLA-C, HNRNPA1, HNRNPUL1, HSPA1A/HSPA1B, IGF2R, IST1 (includes EG:307833), KANSL2, KIFC3, LCK, MAP3K4, MCM3AP, MED23, MGA, MYH14, NAPA, NEFM, NISCH, NUMA1, PACS1, PJA2, PLEKHH3, PLOD1, PLXNA3, PNPT1, PPM1G, PRDX6, PRPF31, PTPRA, PTPRE, PTPRF, RAB11FIP3, RAD21, RNF213, RNF34, RPLP0P2, SBF1, SEPT1, SLC7A5, SORBS2, TAOK1, THAP7, TRIP12, VIM, VPS72 (includes EG:100001285), ZNF7 in a patient comprising the step of detecting antibodies binding said marker proteins, detecting said marker proteins or antigenic fragments thereof in a sample of the patient.

2. Method of diagnosing breast cancer in a patient by detecting at least 2 or least 20 % of the marker proteins selected from the markers of List 3, 4, 5, 6, 7, 8, 9, 10, 11, 12, 13, 14, 15, 16, 17, 18, 19, 20 or any combination thereof in a patient comprising the step of detecting antibodies binding said marker proteins, detecting said marker proteins or antigenic fragments thereof in a sample of the patient.

3. Method of diagnosing breast cancer in a patient by detecting a marker protein selected from any one of List 1 in a patient comprising the step of detecting antibodies binding said marker protein, detecting said marker protein or antigenic fragments thereof in a sample of the patient.

4. The method of claim 2 wherein the combination is of lists 3, 4, 5 and 6, wherein the markers are selected from ARID5A, ATAD3A/ATAD3B, ATP5SL, BCL3, BSDC1, C12orf32, C1orf144, CCDC64, CDK11A/CDK11B, CDK5RAP2, CDT1, CELF5, CHKB, CTBP2, DDX19B, DDX24, E4F1, EFR3A, EIF3G, FAM171B, FAM208A, FIS1 (includes EG:288584), FKBP8, FURIN, GIPC1, GMFG, GTF2IRD1, HADH, HNRNPA1, HNRNPF, HNRNPUL1, HSPG2, HTT, ISG15, IST1 (includes EG:307833), KANSL2, KIF22, LCP1, MAGED2, MAPKAPK2, MED12, MED23, MEGF8, MGA, MKI67IP, MTUS1, MYH14, MYO9B, NARG2, NEDD4L, NFKBID, NOP58, NU-MA1, OS9, P4HTM (includes EG:301008), PHACTR2, PIN1, PNPT1, PPM1G, PRPF31, PSMB8, RAB11FIP3, RAD21, RASGRP2, RRBP1, RSPH10B/RSPH10B2, SCAP, SERBP1, SETD4, SNRNP35, SORBS2, STX18, STXBP3, TAOK1, TNXB, TPM3, TUBA1B, TXN2, UBA1, USP12, VIM, VPS8 (includes EG:209018), VTI1B, ZC3H7A, ZC3H7B.

5. The method of claim 2 wherein the combination is of lists 7, 8, 9, 10, 11, and 12 wherein the markers are selected from AARS, ABAT, ACAT2, ACIN1, ACOT9, ACTN4, ACTR3, ADAMTS10, ADAMTS16, ADH5 (includes EG:100145871), AEBP1, AFMID, AGAP6 (includes others), AKR1C4, AKT1, AMBRA1, AMN1 (includes EG:196394), AMT, ANTXR1, ANXA6, AP2A1, APBB1, APBB1IP, APOH, ARCN1, ARHGAP1, ARHGAP44, ARHGEF11, ATIC, ATN1, ATP5SL, ATRX, ATXN1, AURKAIP1, BAZ1B, BCAM, BCL11A, BEX4, BIN1, BOD1L1, BRPF1, C12orf35, C12orf45, C8orf37, CACNB3, CAMSAP1, CCDC94, CCND1, CCT5, CD81, CD97, CDC25B, CDK13, CDT1, CENPF, CEP70, CHD3, CHMP1B, CINP, CISH, CLCN2, CLTC, CLU, CORO2B, CPE, CPNE1, CPNE6, CRAT, CSRNP1, CTBP2, CTSH, CYLD, DDX24, DDX39A, DENND1C, DFFA, DNAJC10, DOK2, E4F1, EEF1D, EEFSEC, EGR1, EHD4, EIF3G, EIF3J, EIF3L, EZR, FAM134A, FAM208A, FBRS, FBXL5, FBXO34, FBXO6, FCHO1, FGB, FIS1 (includes EG:288584), FLII, FLNA, FNTB, FOXL1, FTL, FUT8, GABARAPL2, GART, GBAS, GDAP1L1, GGA3, GLUL, GMPPB, GNAI2, GOLM1, GTF2IRD1, HARS, HIVEP1, HLA-A, HLA-B, HLA-C, HMGB2, HNRNPA1, HNRNPF, HNRNPUL1, HOOK2, HSP90AB1, HSPA1A/HSPA1B, HSPB1, HSPG2, HUWE1, IGF2R, IRF1 (includes EG:16362), IST1 (includes EG:307833), KANSL2, KAT7, KHDRBS1, KIF22, KLHDC3, KPNB1, KRT8, LAMC1, LCK, LRP1 (includes EG:16971), LRRC47, LRSAM1, LUC7L3, MAF1 (includes EG:315093), MAGED2, MAP3K3, MAP3K4, MAPK8IP2, MAPK8IP3, MCM3AP, MCM7, MED23, MGA, MGEA5, MINK1, MMP14, MTA2, MUC2 (includes EG:4583), MYH14, MYO9B, NAGLU, NAP1L1, NDC80, NEDD4L, NEFM, NISCH, NLE1, NOC2L, NONO, NOP58, NPM1, NRXN2, NT5C3L, NUMA1, NUP85 (includes EG:287830), OLFM2, OS9, OTUB1, PACS1, PCDH7, PCDH9, PDCD6IP, PDXK, PFAS, PITPNC1, PJA2, PLCE1, PLCG1, PLEKHH3, PLXNA1, PMS2, PNPT1, POGK, POGZ, POLR2H, PPIP5K1, PPP2R5D, PRDX1, PREPL, PRKD2, PRPF31, PRRC2C, PRRT1, PSMC4, PSMD13, PTK2B, PTPN23, PTPN7, PTPRA, PTPRF, PXN, QARS, RAB11FIP3, RAB5C, RABEPK, RAC2, RAD21, RAD9A, RAN, RARS, RBM25, RBM5, RGS2 (includes EG:19735), RNF146, RNF213, RPL10, RPLP0P2, RPS17/RPS17L, RPS18, RPS6KB2, RRBP1, RSPH10B/RSPH10B2, SAMD14, SBF1, SCAP, SCG5, SCO1, SDCBP, SEC13, SEPT1, SEPT6, SERBP1, SF3A3, SH3BGRL3, SH3BP1, SLC25A6, SLC7A5, SND1, SNRNP200, SNRNP35, SNX4, SNX5, SNX6, SORBS2, SPAG9, SPNS2, SPTBN1, SRA1, SRPR, STAB1, STAT1, STIP1, STMN4, STRN4, STX18, STXBP3, SUPT5H, TAF4B, TAOK1, TAX1BP1, THAP7, TIAF1, TMOD3, TMSB10/TMSB4X, TNFSF13, TPM2, TPM3, TPR, TRADD, TRIP12, TSC2, TSEN2, TTYH1, TXLNA, TXLNG2P, TYK2, U2AF1, UBA52, UBE2B, UBR4, UBR5, UQCRC1, URI1, USP12, VASP, VAV2, VIM, VPS37B, VPS72 (includes EG:100001285), WARS, WDR47, WDR6, WDTC1, ZC3H13, ZC3H7A, ZFP14, ZFP2, ZFYVE28, ZNF317, ZNF7, ZNFX1.

6. The method of claim 2 wherein the combination is of the markers are selected from lists 13, 14, 15, and 16 wherein the markers are selected from A2M, ABCA11P, ABI1, ACTR1B, AIDA, AKAP9, AKR1C4, AMICA1, ANXA11, APEX1, ARAP1, ARCN1, ARF5, ARHGEF40, ARID4B, ARL8A, ATG16L1, ATP5D, ATP5SL, ATXN2, BCAM, BZRAP1, BZW2, C12orf45, C17orf56, C17orf70, C19orf25, C21orf2, CCDC149, CCL28, CCND1, CDC25B, CDC37, CECR5, CEP57, CHD1L, CIC, CNTD1, CNTROB, COL3A1, CPNE1, CSF1R, CSRNP1, CTSD, CUEDC2, CUL1, DBNL, DHX16, DHX9, DMPK, EGR1, EIF4G1, ERP29, EVPL, EZR, FAM192A, FIGNL1, FLNA, FLOT1, FOXP3, FUT8, FZR1, GABARAPL2, GLYR1, GNAS, GOLGA2, GOLGA7, GON4L, HK1, HMGB2, HMOX2, HNRNPA3, HNRNPUL1, HTATIP2, IGLC1, IRF1 (includes EG:16362), ITFG3, KCNJ14, KHDRBS1, KIAA1244, KLF5, LARP1, LCMT2, LOC440354, LPXN, LRRC47, LSP1 (includes EG:16985), MAGED2, MAPRE1, MCM5, MEGF8, METTL3, MICAL3, MORF4L1, MYO9A, NAP1L1, NCOA4, NEDD4L, NRG3, NXPH3, P4HB, PACS1, PARD3, PCGF2, PDZD4, PEF1, PIP5K1A, PKP3, PLCB3, POLI, POLR2H, POLR2L, POLR3E, POTEE/POTEF, PPM1G, PRKCD, PSMB3, PSME4, R3HDM4, RAB11FIP3, RASGRP1, RCBTB1, RCOR2, RGS2 (includes EG:19735), RIPK1, RNF34, RRBP1, RRM1, RSL24D1, SAFB2, SAMD14, SAP18, SFN, SIRT2, SLC7A5, SMAP2, SMCR8, SND1, SNX5, SPHK2, SPOCK2, SSBP2, STAT4, STAT6, STK10, SURF4, SURF6, TAF7, TALDO1, THAP7, THOP1, TNFRSF6B, TNRC18, TRAFD1, TRIM44, TSC2, TXLNG2P, TXNDC5, UNC45A, UTY, VPS72 (includes EG:100001285), WAC, WASH1/WASH5P, WDR33, WDR47, WDR73, XRCC1 (includes EG:22594), ZNF202, ZNF638, ZNF839.

7. The method of claim 2 wherein the combination from lists 17, and 18 wherein the markers are selected from ANXA6, ATIC, BAZ1B, BIN1, BIN3, CBX4, CCDC51, CCNL2, CDC37, CUL7, DCAF6, DSP, EHMT1, ENTPD6, ETFA, EXOSC10, EZH1, HEXDC, HMGN2, HSP90B1, IGHG1, IK, ILF3, IMMT, IMP4, KAT2A, KCMF1, KIFC3, LDHB (includes EG:3945), LOC284023, LRBA, LTBP3, MYCL1, NAPA, NEFL, NEU1, NUDT16L1, PACS1, PJA2, PLBD1, PLCB3, PLEKHA4, PLEKHO1, PLOD1, PLXNA3, PPID, PPIF, PPP1R9B, PRDX6, PSMB6, PTPRE, RCL1, RECQL4, RNF146, RPL37A, RPL7, SCAF1, SMARCC2, STX11, SWI5, TRO, TSC22D3, VIMP, WDR75.

8. The method of claim 2 wherein the combination is of lists 19 and 20 wherein the markers are selected from ACAT2, ACTN4, ADAMTS10, AGAP2, AIP, AKAP17A, AMBRA1, ANGPTL2, ANXA6, AP2M1, ARHGAP44, ARL4D, BCL11A, BIN3, BNIP1, BTBD7, C3orf19, CANX, CDT1, CELSR1, CORO2B, CTSH, D2HGDH, DFFA, EHMT1, EIF3G, EIF4B, FAM69B, FLII, GLUL, HIST3H2A, HLA-C, HLA-C, HNRNPA0, HNRNPH1, HNRNPK, HNRPDL, HSPA1A/HSPA1B, HSPA9, IGF2R, IK, IK, INPPL1, KCNK5, KIFC3, LCK, LGALS3, LONP1, MAP3K4, MCM3AP, METTL17, MSLN, MTA2, NAPA, NEFM, NISCH, NOC2L, NRBP2, NUP93, PA2G4, PCDH9, PDE2A, PGD, PGRMC1, PLEKHH3, PLOD1, PLXNA3, POLR2B, PRDX6, PTPRA, PTPRE, PTPRF, PUF60, PUF60, PXN, RBM10, RNF213, RNF219, RNF34, RPLP0P2, RSPRY1, SAT2, SBF1, SEPT1, SLC7A5, SLU7 (includes EG:10569), SPTAN1, SSRP1, ST3GAL3, STOML2, TBC1D9B, TF, TRIP12, TSEN2, TTC3, ZNF132, ZNF300, ZNF431, ZNF7, ZSWIM4.

9. The method of any one of claims 1 to 8 comprising detecting breast cancer and benign tumors when distinguishing healthy conditions vs. benign tumor, healthy conditions vs. malign tumor, benign tumor vs. malign tumor, healthy conditions vs. hereditary breast cancer, and malign tumor vs. hereditary breast cancer, preferably wherein the markers according to claim 4 are used for distinguishing healthy conditions vs. benign tumor, the markers according to claim 5 are used for healthy conditions vs. malign tumor, the markers according to claim 6 are used for distinguishing benign tumor vs. malign tumor, the markers according to claim 7 are used for distinguishing healthy conditions vs. hereditary breast cancer, and the markers according to claim 8 are used for distinguishing malign tumor vs. hereditary breast cancer, and/or preferably wherein a positive result in distinguishing said indications can prompt a further cancer test, in particular more invasive tests than a blood test such as a biopsy.

10. The method of any one of claims 1 to 9, wherein the step of detecting antibodies binding said marker proteins, detecting said marker proteins or antigenic fragments thereof comprises comparing said detection signal with detection signals of a healthy control and comparing said detection signals, wherein an increase in the detection signal indicates breast cancer.

11. The method of any one of claims 1 to 10, a) wherein the step of detecting antibodies binding said marker proteins, detecting said marker proteins or antigenic fragments thereof comprises comparing said detection signal with detection signals of one or more known control samples of conditions selected from cancer, wherein the control signals are used to obtain a marker dependent signal pattern as indication classifier and the marker dependent signals of the patient is compared with and/or fitted onto said pattern thereby obtaining information of the diagnosed condition.

12. The method of any one of claims 1 to 11, a) wherein the step of detecting antibodies binding said marker proteins, detecting said marker proteins or antigenic fragments thereof comprises comparing said detection signal with detection signals of a cancerous control and comparing said detection signals, wherein a detection signal from the sample of the a patient in amplitude of at least 60%, preferably at least 80%, of the cancerous control indicates breast cancer; or b) wherein a detection signal in at least 60%, preferably at least 75%, of the used markers indicates breast cancer.

13. The method of treating a patient comprising breast cancer, comprising detecting cancer according to any one of claims 1 to 12 and removing said breast cancer.

14. A kit of diagnostic agents suitable to detect any marker or marker combination as defined in claims 1 to 8, preferably wherein said diagnostic agents comprise marker proteins or antigenic fragments thereof suitable to bind antibodies in a sample, especially preferred wherein said diagnostic agents are immobilized on a solid support,
optionally further comprising a computer-readable medium or a computer program product, comprising signal data for control samples with known conditions selected from cancer, and/or calibration or training data for analysing said markers provided in the kit for diagnosing breast cancer or distinguishing conditions selected from healthy conditions, cancer.

15. The kit of claim 14 comprising at most 3000 diagnostic agents, preferably at most 2500 diagnostic agents, at most 2000 diagnostic agents, at most 1500 diagnostic agents, at most 1200 diagnostic agents, at most 1000 diagnostic agents, at most 800 diagnostic agents, at most 500 diagnostic agents, at most 300 diagnostic agents, at most 200 diagnostic agents, at most 100 diagnostic agents.
